# EUROPEAN PATENT SPECIFICATION

(11) **EP 2 419 142 B1**
(45) Date of publication and mention of the grant of the patent: **15.01.2020**
(21) Application number: 10717257.9
(22) Date of filing: 12.04.2010
(51) Int. Cl.: A61K 51/04, A61K 47/68, B82Y 5/00

(54) **Pretargeting kit, method and agents used therein**
Kit für Pretargeting, Verfahren und darin verwendete Mittel
Kit de pré-ciblage, procédé et agents utilisés avec celui-ci

(30) Priority: 16.04.2009 EP 09158058; 29.10.2009 EP 09174489
(43) Date of publication of application: 22.02.2012
(73) Proprietor: Tagworks Pharmaceuticals B.V., 5611 NX Eindhoven (NL)
(72) Inventor: ROBILLARD, Marc, S., NL-5656 AE Eindhoven (NL); ROSSIN, Raffaella, NL-5656 AE Eindhoven (NL); LUB, Johan, NL-5656 AE Eindhoven (NL); RENART VERKERK, Pascal, NL-5656 AE Eindhoven (NL); BURDINSKI, Dirk, NL-5656 AE Eindhoven (NL)
(74) Representative: V.O.
(86) International application number: PCT/IB2010/051565
(87) International publication number: WO 2010/119389

(56) References cited:
- WO-A-2007/039858
- WO-A2-2010/051530
- BLACKMAN M L ET AL: "Tetrazine ligation: Fast bioconjugation based on inverse-electron-demand Diels-Alder reactivity" JOURNAL OF THE AMERICAN CHEMICAL SOCIETY, vol. 130, no. 41, 15 October 2008 (2008-10-15), pages 13518-13519, XP002544618 ISSN: 0002-7863 cited in the application
- THALHAMMER F ET AL: "The reactivity of simple open chain and cyclic dienophiles in Diels-Alder-reactions with inverse electronic supply" TETRAHEDRON LETTERS, vol. 31, no. 47, 1990, pages 6851-6854, XP002544619 ISSN: 0040-4039
- DEVARAJ, NEAL K. ET AL: "Tetrazine -Based Cycloadditions: Application to Pretargeted Live Cell Imaging" BIOCONJUGATE CHEMISTRY, vol. 19, no. 12, 2008, pages 2297-2299, XP002544620 ISSN: 1043-1802
- DATABASE CAPLUS [Online] CHEMICAL ABSTRACTS SERVICE, COLUMBUS, OHIO, US; BLACKMAN, MELISSA LYNN ET AL: "A bioorthogonal reaction based on an inverse electron demand Diels-Alder reaction" XP002544621 retrieved from STN Database accession no. 2008:954676 & ABSTRACTS OF PAPERS, 236TH ACS NATIONAL MEETING, PHILADELPHIA, PA, UNITED STATES, AUGUST 17-21, 2008 , ORGN-646 PUBLISHER: AMERICAN CHEMICAL SOCIETY, WASHINGTON, D. C. CODEN: 69KXQ2, 2008,
- XINGHAI NING ET AL: "Visualizing metabolically labeled glycoconjugates of living cells by copper-free and fast huisgen cycloadditions", ANGEWANDTE CHEMIE (INTERNATIONAL EDITION), vol. 47, no. 12, 7 March 2008 (2008-03-07) , pages 2253-2255, XP002659161, ISSN: 1521-3773, DOI: 10.1002/ANIE.200705456 [retrieved on 2008-02-14]

## Description

### FIELD OF THE INVENTION

The invention relates to a pretargeting method, for targeted medical imaging and/or therapeutics, wherein use is made of abiotic reactive chemical groups that exhibit bio-orthogonal reactivity towards each other. The invention also relates to a pretargeting kit comprising at least one Pre-targeting Probe and at least one Effector Probe, wherein the Pre-targeting Probe comprises a primary targeting moiety and a first Bio-orthogonal Reactive Group, and wherein the Effector Probe comprises an Effector Moiety, such as a label or a pharmaceutically active compound, and a second Bio-orthogonal Reactive Group. The invention also relates to pre-targeting agents used in the above-mentioned method and kit. The invention particularly pertains to nuclear imaging and radiotherapy.

### BACKGROUND OF THE INVENTION

In many areas of medical diagnosis and therapy, it is desired to selectively deliver an agent, such as a therapeutic agent (a drug) or a diagnostic (e.g. imaging) agent, to a specific site, or a confined region, in the body of a subject such as a patient.

Active targeting of an organ or a tissue is achieved by the direct or indirect conjugation of the desired active moieties (e.g. a contrast enhancing agent or a cytotoxic compound) to a targeting construct, which binds to cell surfaces or promotes cellular uptake at or the target site of interest. The targeting moieties used to target such agents are typically constructs that have affinity for cell surface targets (e.g., membrane receptors), structural proteins (e.g., amyloid plaques), or intracellular targets (e.g., RNA, DNA, enzymes, cell signaling pathways). These moieties can be antibodies (fragments), proteins, aptamers, oligopeptides, oligonucleotides, oligosaccharides, as well as peptides, peptoids and organic drug compounds known to accumulate at a particular disease or malfunction. Alternatively, a contrast/therapeutic agent may target a metabolic pathway, which is upregulated during a disease (like infection or cancer) such as DNA, protein, and membrane synthesis and carbohydrate uptake. In diseased tissues, abovementioned markers can discriminate diseased cells from healthy tissue and offer unique possibilities for early detection, specific diagnosis and (targeted) therapy.

An important criterion for successful molecular imaging/therapy agents in general and nuclear imaging/therapy agents in particular is that they exhibit a high target uptake while showing a rapid clearance (through renal and/or hepatobiliary systems) from non-target tissue and from the blood. However, this is often problematic: for example, imaging studies in humans have shown that the maximum concentration of a radiolabeled antibody at the tumor site is attainable within 24 h but several more days are required before the concentration of the labeled antibody in circulation decreases to levels low enough for successful imaging to take place.

These problems (especially for nuclear imaging and therapy) with slow or insufficient accumulation in target tissue and slow clearance from non-target areas have led to the application of pre-targeting approaches.

Pretargeting refers to a step in a targeting method, wherein a primary target (e.g. a cell surface) is provided with a Pre-targeting Probe. The latter comprises a secondary target, which will eventually be targeted by a further probe (the Effector Probe) equipped with a secondary targeting moiety.

Thus, in pre-targeting, a Pre-targeting Probe is bound to a primary target. The Pre-targeting Probe also carries secondary targets, which facilitate specific conjugation to a diagnostic (imaging) and/or therapeutic agent, the Effector Probe. After the construct forming the Pre-targeting Probe has localized at the target site (taking time, e.g. 24 h), a clearing agent can be used to remove excess from the blood, if natural clearance is not sufficient. In a second incubation step (preferably taking a shorter time, e.g., 1-6 hours), the Effector Probe binds to the (pre)bound Pre-targeting Probe via its secondary targeting moiety. The secondary target (present on the Pre-targeting Probe) and the secondary targeting moiety (present on the Effector Probe) should bind rapidly, with high specificity and high affinity and should be stable within the body.

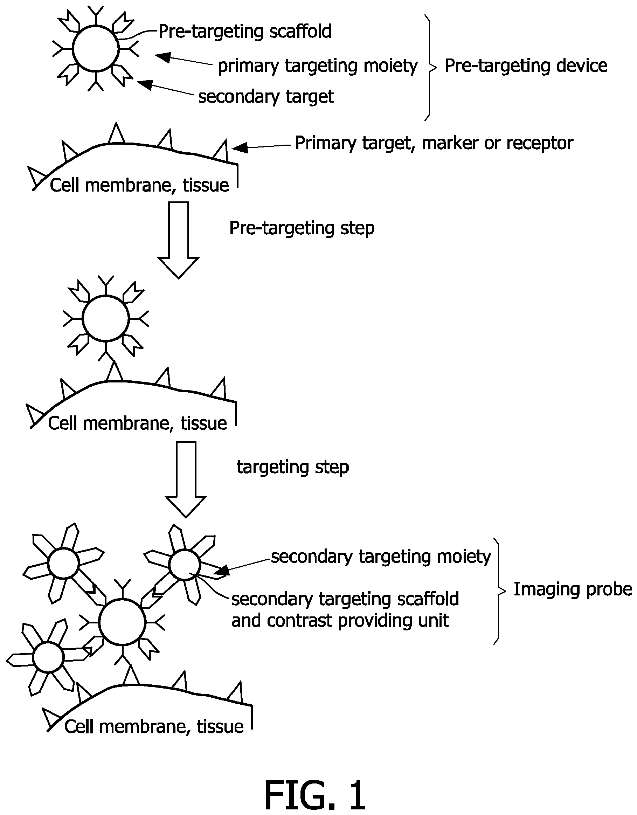

The general concept of pre-targeting is outlined for imaging in Fig. 1. Herein the Effector Probe is an imaging probe comprising a detectable label for an imaging modality. The Effector Probe binds to the (pre)-bound Pre-targeting Probe via its secondary targeting groups.

Common examples for secondary target/secondary targeting moiety pairs are biotin/streptavidin or antibody/antigen systems. To be effective, the Effector Probe must be rapidly excreted from the body (e.g., through the kidneys) to provide the desired high tumor accumulation with relatively low non-target accumulation. Therefore, these probes are usually small.

In nuclear imaging and radiotherapy the concept of pre-targeting is of further advantage, as the time consuming pre-targeting step can be carried out without using radionuclides, while the secondary targeting step using a radionuclide can be carried out faster. The latter allows the use of shorter lived radionuclides with the advantage of minimizing the radiation dose to the patient and, for instance, the usage of PET agents instead of SPECT agents. Furthermore, in general, this approach facilitates the usage of a universal contrast agent.

The entities that carry out highly selective interactions in biology in general (like antibody-antigen), and in pre-targeting in particular (biotin-streptavidin, antibody/haptens, antisense oligonucleotides), are very large. As a result, pre-targeting with peptides and small organic moieties as primary targeting groups, as well as metabolic imaging and intracellular target imaging, have remained out of reach as the size of the secondary targets makes the use of small primary groups pointless.

Moreover, the current pretargeting systems are hampered by factors associated with their biological nature. Biotin is an endogenous molecule and its conjugates can be cleaved by the serum enzyme biotinidase. When antisense pre-targeting is used, the oligonucleotides can be subject to attack by RNAse and DNAse. Proteins and peptides are also subject to natural decomposition pathways. These interactions can be further impaired by their non-covalent and dynamic nature and limited on-target residence time. As a result, the time between the addition of the two components in pre-targeting is limited, which may lead to suboptimal target to non-target ratios. Also, endogenous biotin competes with biotin conjugates for streptavidin binding. Finally, streptavidin is highly immunogenic.

A recent development is to avoid the drawbacks associated with pretargeting solely on the basis of natural/biological targeting constructs (i.e., biotin/streptavidin, antibody/hapten, antisense oligonucleotides).

In this respect reference can be made to WO 2006/038185 as a disclosure of a pretargeting method, for targeted medical imaging and/or therapeutics, wherein use is made of abiotic reactive chemical groups that exhibit bio-orthogonal reactivity towards each other. In this reference, the bio-orthogonally reacting groups are the reaction partners in a Staudinger ligation, i.e. an azide and a phoshine. The described selection of the Staudinger ligation as the coupling chemistry in pretargeting results in the availability of reactive partners that are abiotic, that form a stable adduct under physiological conditions, and that recognize only each other, while ignoring their cellular/physiological surroundings (i.e. they are bio-orthogonal).

Another reference on the use, in a pretargeting method, of abiotic reactive chemical groups that exhibit bio-orthogonal reactivity towards each other, is WO 2007/039858. Herein the bio-orthogonal reactive groups are the reaction partners in a [3+2] azide - alkyne cycloaddition.

Another type of coupling chemistry is described by Neal K. Devaraj, Ralph Weissleder, and Scott Hilderbrand in Bioconjugate Chem. 2008, 19, 2297-2299. This relates to the application of bioorthogonal tetrazine cycloadditions to live cell labeling. The reaction partners herein are 3-(p-benzylamino)-1,2,4,5-tetrazine and a norbornene, viz. (1S,2S,4S)-bicyclo[2,2,1]hept-5-en-2-yl acetic acid, which undergo a Diels-Alder cycloaddition followed by a retro Diels-Alder reaction, in which dinitrogen (N₂) is released. This coupling chemistry is also referred to as an inverse electron-demand Diels-Alder reaction. Reference is made to the pretargeting of human breast cancer SKBR3 cells by the monoclonal antibody trastuzumab (Herceptin) labeled with norbornene, followed by tagging the cells with tetrazine linked with the near IR fluroescent probe VT680.

The foregoing types of coupling chemistry, although useful, are subject to further improvement. This, *inter alia,* in the sense that relatively high concentrations of reactants are needed. Particularly, this means that the Effector Probes, in order to sufficiently bind to Pre-targeting Probes, require a relatively long circulation time and/or high concentration. It is desired to be able to lower the necessary concentration of Effector Probes, whilst retaining the advantages of the aforementioned bio-orthogonal pre-targeting method.

Further, particularly with reference to application in nuclear imaging and radiotherapy it is desired to present a fast and efficient bio-orthogonal coupling chemistry.

### SUMMARY OF THE INVENTION

In order to better address the foregoing desires, the invention, in one aspect, provides a kit for targeted medical imaging and/or therapeutics, comprising at least one Pre-targeting Probe and at least one Effector Probe, wherein the Pre-targeting Probe comprises a Primary Targeting Moiety and a first Bio-orthogonal Reactive Group, and wherein the Effector Probe comprises an Effector Moiety, such as a label or a pharmaceutically active compound, and a second Bio-orthogonal Reactive Group, wherein either of the first and second Bio-orthogonal Reactive Groups is a dienophile and the other of the first and second Bio-orthogonal Reactive Groups is a diene, wherein the dienophile is an 8-member ring dienophile satisfying formula (1): 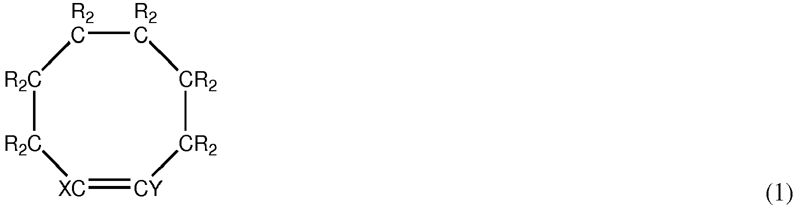 wherein each R independently denotes H, or, in at most six instances, a substituent selected from the group consisting of alkyl, O-alkyl, S-alkyl, F, Cl, Br, I, SO₂, NO₂, NR'R" with R' and R" each independently being H or alkyl, C(=O)Oalkyl, C(=O)Oaryl, CONR'R" with R' and R" each independently being H, aryl or alkyl, OCOalkyl, OCOaryl, NR'COalkyl with R' being H or alkyl, NR'COaryl with R' being or alkyl, NR'C(=O)Oalkyl with R' being H or alkyl, NR'C(=O)Oaryl with R' being H or alkyl, OCONR'alkyl with R' being H or alkyl, OCONR'aryl with R' being H or alkyl, NR'CONR"alkyl with R' and R" each independently being H or alkyl, NR'CONR"aryl with R' and R" each independently being H or alkyl, NR'CSNR"alkyl with R' and R" each independently being H or alkyl, and NR'CSNR"aryl with R' and R" each independently being H or alkyl; with at least one R comprised in a linker moiety, optionally via a spacer, to the Pre-targeting Probe or the Effector Probe, and wherein X and Y each independently denote H, or a substituent selected from the group consisting of alkyl, O-alkyl, S-alkyl, F, Cl, Br, I, SO₂, NO₂, and NRR' with R and R' each independently being H or alkyl, or together form a bond; and wherein the diene is selected so as to be capable of reacting with the dienophile by undergoing a Diels-Alder cycloaddition followed by a retro Diels-Alder reaction, and wherein the diene satisfies the formula

(7)

wherein R¹ and R² each independently denote a substituent selected from the group consisting of 2-pyridyl, phenyl, or phenyl substituted with one or more electron-withdrawing groups such as NO₂, CN, COOH, COOR, CONH₂, CONHR, CONR₂, CHO, COR, SO₂R, SO₂OR, NO, and Ar, wherein R is C₁-C₆ alkyl and Ar stands for an aromatic group, particularly phenyl, pyridyl, or naphthyl.

In another aspect, the invention provides a pre-targeting method, as well as pre-targeting agents used therein, and targeted medical imaging or therapy wherein this kit is used.

In a still further aspect, the invention is a compound satisfying formula (1), for use in a pre-targeting method in an animal or a human being.

### DETAILED DESCRIPTION OF THE INVENTION

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

It is furthermore to be noticed that the term "comprising", used in the description and in the claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

In several chemical formulae reference is made to "alkyl" and "aryl." In this respect "alkyl", each independently, indicates an aliphatic, straight, branched or cyclic alkyl group of up to ten carbon atoms, possible including 1-3 heteroatoms such as O, N, or S, and preferably of 1-6 carbon atoms and "aryl," each independently, indicates an aromatic or heteroaromatic group of up to ten carbon atoms, possibly including 1-3 heteroatoms such as N or S. In several formulae, groups or substituents are indicated with reference to letter ssuch as "A", "B", "X", "Y", and various numbered "R" groups. The definitions of these letters are to be read with reference to each formula, i.e. in different formulae, these letters, each indendepently can have different meanings unless indicated otherwise.

### BRIEF DESCRIPTION OF THE DRAWINGS

Fig. 1 depicts a general scheme of a pretargeting concept, as discussed above;

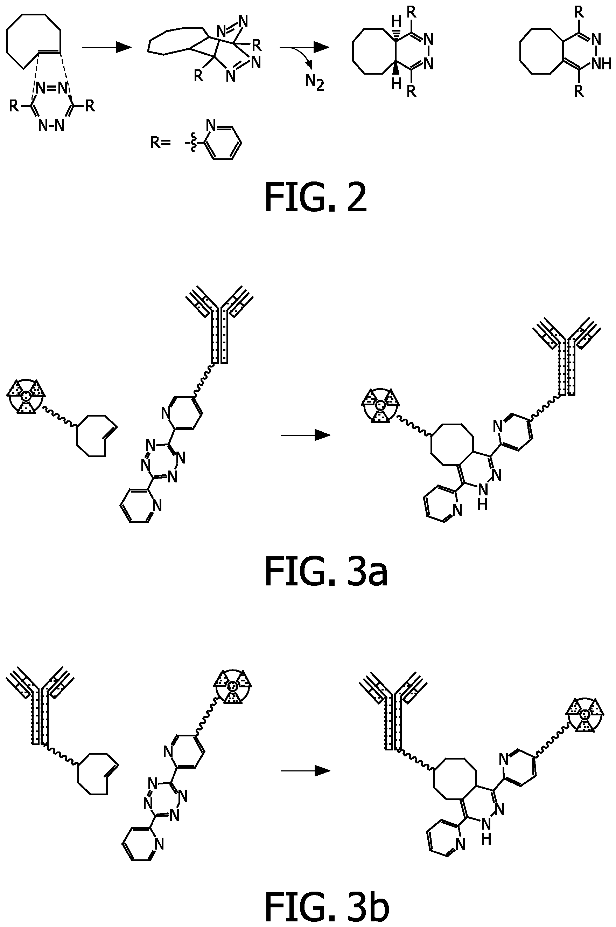

Fig.2. provides the reaction scheme for a [4+2] Diels-Alder reaction; between (3,6)-di-(2-pyridyl)-s-tetrazine and E-cyclooctene followed by a retro Diels Alder reaction in which the product and nitrogen is formed. Because the trans cyclooctene derivative doesn't contain electron withdrawing groups as in the classical Diels Alder reaction, this type of Diels Alder reaction is distinguished from the classical one, and frequently referred to as an "inverse electron demand Diels Alder reaction". In the following text the sequence of both reaction steps, i.e. the initial Diels-Alder cyclo-addition (typically an inverse electron demand Diels Alder cyclo-addition) and the subsequent retro Diels Alder reaction will be referred to in shorthand as "retro Diels Alder reaction."
Fig. 3 (a and b) depicts general schemes for pre-targeting using retro Diels-Alder chemistry;
Fig. 4 to Fig. 7 illustrate synthesis schemes for compounds used in the Examples.
Fig. 8-9 illustrate synthesis schemes for compounds used in the in vivo example.
Fig 10-12 illustrate the in vivo feasibility of the present invention.

### DETAILED DESCRIPTION OF THE EMBODIMENTS

### Retro Diels-Alder reaction

The Retro Diels-Alder coupling chemistry generally involves a pair of reactants that couple to form an unstable intermediate, which intermediate eliminates a small molecule (depending on the starting compounds this may be e.g. N₂, CO₂, RCN, as the sole by-product through a retro Diels-Alder reaction to form a stable product. The paired reactants comprise, as one reactant (i.e. one Bio-orthogonal Reactive Group), a a derivative of tetrazine, and, as the other reactant (i.e. the other Bio-orthogonal Reactive Group), a cyclooctene according to formula (1).

The Retro Diels-Alder coupling chemistry generally involves a pair of reactants that couple to form an unstable intermediate, which intermediate rearranges to a stable adduct through a retro Diels-Alder reaction. The paired reactants comprise, as one reactant (i.e. one Bio-orthogonal Reactive Group), a diene such as an electron-deficient tetrazine, or the like, and, as the other reactant (i.e. the other Bio-orthogonal Reactive Group), a strained cyclooctene according to formula (1).

The exceptionally fast reaction of, e.g., electron-deficient (substituted) tetrazines with e.g. strained E-cyclooctene results in a ligation intermediate that rearranges to a stable dihydropyridazine by eliminating N₂ as the sole by-product in a [4+2] Retro Diels-Alder cycloaddition. This is shown in Fig. 2.

The two reactive species are abiotic and thus do not undergo a fast metabolism in vivo. They are bio-orthogonal, e.g. they selectively react with each other in physiologic media.

References on the Inverse electron demand Diels Alder reaction, and the behavior of the pair of reactive species include: Thalhammer, F; Wallfahrer, U; Sauer, J, Tetrahedron Letters, 1990, 31 (47), 6851-6854; Wijnen, JW; Zavarise, S; Engberts, JBFN, Journal Of Organic Chemistry, 1996, 61, 2001-2005; Blackman, ML; Royzen, M; Fox, JM, Journal Of The American Chemical Society, 2008, 130 (41), 13518-19).

It will be understood that, in a broad sense, according to the invention the aforementioned coupling chemistry can be applied to basically any pair of molecules, groups, or moieties that are capable of being used in pretargeting. I.e. one of such a pair will comprise a primary targeting moiety, that is capable of binding to a primary target, and further comprises at least one secondary target. The other one will be a secondary targeting moiety suitable for use in binding to said secondary target, and further comprises a moiety suitable for exerting therapeutic action (typically a pharmaceutically active compound), or for being addressed by an imaging technique (i.e. a label), or both.

Thus, according to the invention, either of the Pre-targeting Probe and the Effector Probe is functionalized with a cyclooctene, and the other is functionalized with a tetrazine. This is illustrated in Fig. 3. The scheme on top (Fig. 3a) indicates a Pre-targeting Probe comprising di-pyridyl tetrazine linked, via a linker moiety (preferably comprising a flexible spacer) to an antibody as the primary targeting moiety, and an Effector Probe comprising cyclooctene (as the secondary targeting moiety) attached, via a linker (a flexible spacer), to a detectable label. The scheme below (Fig. 3b) shows exactly the opposite, viz. a Pre-targeting Probe comprising the cyclooctene and an Effector Probe comprising the tetrazine.

The person skilled in the art is aware of the wealth of dienes that are reactive in the Retro Diels-Alder reaction. The dienes of the invention are given below, with reference to formula (7).

Particularly useful tetrazine derivatives are electron-deficient tetrazines, i.e. tetrazines substituted with groups or moieties that do not generally hold as electron-donating, and preferably carrying electron-withdrawing substituents.

These electron-deficient tetrazines satisfy the following structural formula:

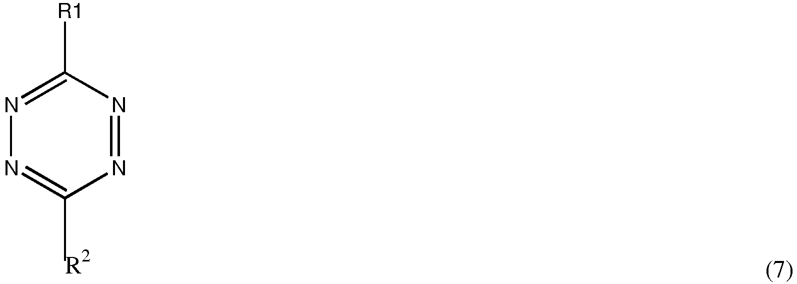

Herein R¹ and R² each independently denote a substituent selected from the group consisting of 2-pyridyl, phenyl, or phenyl substituted with one or more electron-withdrawing groups such as NO₂, CN, COOH, COOR, CONH₂, CONHR, CONR₂, CHO, COR, SO₂R, SO₂OR, NO, Ar, wherein R is C₁-C₆ alkyl and Ar stands for an aromatic group, particularly phenyl, pyridyl, or naphthyl.

In the compounds according to formula (7), the R¹ and R² groups, can further be provided with suitable linker or spacer moieties as discussed below. Analogously, and independently thereof, also the dienophile of formula (1) can further be provided with suitable linker or spacer moieties as discussed below.

The dienophile preferably is an E-cyclooctene.

An advantage of making use of the [4+2] retro Diels-Alder reaction in a pre-targeting strategy is that both the diene and the cyclooctene or cyclooctyne are abiotic and essentially unreactive toward biomolecules inside or on the surfaces of cells and all other regions like serum etc. Thus, the compounds and the method of the invention can be used in a living cell, tissue or organism. Moreover, the reactive groups are relatively small and can be introduced in biological samples or living organisms without altering the biological size significantly. Using the [4+2] retro Diels-Alder reaction it is possible to bind primary targeting moieties which are large in size, e.g. antibodies, with labels or other molecules using small reaction partners, e.g. tetrazine or cyclooctene. Even more advantageously, primary targeting moieties can be bound which are relatively small, e.g. peptides, with labels or other molecules using (matched) relatively small reaction partners, e.g. tetrazine and cyclooctene. The size and properties of the Pre-targeting Probe and Effector Probe are not greatly affected by the secondary target and secondary targeting moiety, allowing (pre)targeting schemes to be used for small targeting moieties. Because of this, other tissues can be targeted, i.e. the destination of the probes is not limited to the vascular system and interstitial space, as is the case for current pretargeting with antibody-streptavidin. According to one embodiment, the invention is used for targeted imaging.

According to this embodiment, imaging of a specific primary target is achieved by specific binding of the primary targeting moiety of the Pre-targeting Probe and detection of this binding using detectable labels comprised in the Effector Probe.

### Primary Target

A "primary target" as used in the present invention relates to a target to be detected in a diagnostic and/or imaging method, and/or to be modulated, bound, or otherwise addressed by a pharmaceutically active compound, or other therapeutic modality.

The primary target can be selected from any suitable targets within the human or animal body or on a pathogen or parasite, e.g. a group comprising cells such as cell membranes and cell walls, receptors such as cell membrane receptors, intracellular structures such as Golgi bodies or mitochondria, enzymes, receptors, DNA, RNA, viruses or viral particles, antibodies, proteins, carbohydrates, monosaccharides, polysaccharides, cytokines, hormones, steroids, somatostatin receptor, monoamine oxidase, muscarinic receptors, myocardial sympatic nerve system, leukotriene receptors, e.g. on leukocytes, urokinase plasminogen activator receptor (uPAR), folate receptor, apoptosis marker, (anti-) angiogenesis marker, gastrin receptor, dopaminergic system, serotonergic system, GABAergic system, adrenergic system, cholinergic system, opioid receptors, GPIIb/IIIa receptor and other thrombus related receptors, fibrin, calcitonin receptor, tuftsin receptor, integrin receptor, VEGF/EGF receptors, matrix metalloproteinase (MMP), P/E/L-selectin receptor, LDL receptor, P-glycoprotein, neurotensin receptors, neuropeptide receptors, substance P receptors, NK receptor, CCK receptors, sigma receptors, interleukin receptors, herpes simplex virus tyrosine kinase, human tyrosine kinase.

According to a particular embodiment of the present invention, the primary target is a protein such as a receptor. Alternatively, the primary target may be a metabolic pathway, which is upregulated during a disease, e.g. infection or cancer, such as DNA synthesis, protein synthesis, membrane synthesis and carbohydrate uptake. In diseased tissues, above-mentioned markers can differ from healthy tissue and offer unique possibilities for early detection, specific diagnosis and therapy, especially targeted therapy.

### Pre-targeting Probe

A Pre-targeting Probe comprises a moiety that is capable of binding to the primary target of interest.

Targeting moieties are typically constructs that have affinity for cell surface targets (e.g., membrane receptors), structural proteins (e.g., amyloid plaques), or intracellular targets (e.g., RNA, DNA, enzymes, cell signaling pathways). These moieties can be antibodies (fragments), proteins, aptamers, oligopeptides, oligonucleotides, oligosaccharides, as well as peptides, peptoids and organic drug compounds known to accumulate at a particular disease or malfunction.

Particular embodiments of suitable primary targeting moieties for use in the kits of the present invention are described herein and include receptor binding peptides and antibodies. A particular embodiment of the present invention relates to the use of small targeting moieties, such as peptides, so as to obtain a cell-permeable targeting probe.

A "primary targeting moiety" as used in the present invention relates to the part of the targeting probe which binds to a primary target. Particular examples of primary targeting moieties are peptides or proteins which bind to a receptor. Other examples of primary targeting moieties are antibodies or fragments thereof which bind to a cellular compound. Antibodies can be raised to non-proteinaceous compounds as well as to proteins or peptides. Other primary targeting moieties can be made up of aptamers, oligopeptides, oligonucleotides, oligosaccharides, as well as peptoids and organic drug compounds. A primary targeting moiety preferably binds with high specificity, with a high affinity and the bond with the primary target is preferably stable within the body.

In order to allow specific targeting of the above-listed primary targets, the primary targeting moiety of the targeting probe can comprise compounds including but not limited to antibodies, antibody fragments, e.g. Fab2, Fab, scFV, polymers (tumor targeting by virtue of EPR effect), proteins, peptides, e.g. octreotide and derivatives, VIP, MSH, LHRH, chemotactic peptides, bombesin, elastin, peptide mimetics, carbohydrates, monosaccharides, polysaccharides, viruses, drugs, chemotherapeutic agents, receptor agonists and antagonists, cytokines, hormones, steroids. Examples of organic compounds envisaged within the context of the present invention are, or are derived from, estrogens, e.g. estradiol, androgens, progestins, corticosteroids, paclitaxel, etoposide, doxorubricin, methotrexate, folic acid, and cholesterol.

According to a particular embodiment of the present invention, the primary target is a receptor and suitable primary targeting moieties include the ligand of such a receptor or a part thereof which still binds to the receptor, e.g. a receptor binding peptide in the case of receptor binding protein ligands.

Other examples of primary targeting moieties of protein nature include interferons, e.g. alpha, beta, and gamma interferon, interleukins, and protein growth factor, such as tumor growth factor, e.g. alpha, beta tumor growth factor, platelet-derived growth factor (PDGF), uPAR targeting protein, apolipoprotein, LDL, annexin V, endostatin, and angiostatin.

Alternative examples of primary targeting moieties include DNA, RNA, PNA and LNA which are e.g. complementary to the primary target.

According to a particular embodiment of the invention, small lipophilic primary targeting moieties are used which can bind to an intracellular primary target.

According to a further particular embodiment of the invention, the primary target and primary targeting moiety are selected so as to result in the specific or increased targeting of a tissue or disease, such as cancer, an inflammation, an infection, a cardiovascular disease, e.g. thrombus, atherosclerotic lesion, hypoxic site, e.g. stroke, tumor, cardiovascular disorder, brain disorder, apoptosis, angiogenesis, an organ, and reporter gene/enzyme. This can be achieved by selecting primary targets with tissue-, cell- or disease-specific expression. For example, membrane folic acid receptors mediate intracellular accumulation of folate and its analogs, such as methotrexate. Expression is limited in normal tissues, but receptors are overexpressed in various tumor cell types.

According to one embodiment, the Pre-targeting Probe and the Effector Probe can be multimeric compounds, comprising a plurality of primary and/or secondary targets and/or targeting moieties.

The Pre-targeting Probe further comprises the above-mentioned first Bio-orthogonal Reactive group. This group serves as a "secondary target", i.e. as the part of the targeting probe that provides the first reaction partner for the retro Diels-Alder coupling chemistry.

Said secondary target can be either partner of the coupling reaction, as described above. I.e. in one embodiment it is an electron-deficient tetrazine. In another embodiment it is a strained cyclooctene. In the Pre-targeting Probe, the primary targeting moiety and the first Bio-orthogonal Reactive Group can be directly linked to each other. They can also be bound to each other via a linker, and furthermore they can both be linked to a primary targeting scaffold, e.g. a biopolymer such as a polypeptide. Suitable linker moieties include, but are not limited to polyethylene glycol (PEG) chains.

### Effector Probe

An Effector Probe comprises an Effector Moiety that is capable of providing the desired diagnostic, imaging, and/or therapeutic effect. The Effector Probe further comprises a secondary targeting moiety.

The secondary targeting moiety relates to the part of the Effector Probe that forms the reaction partner for the available secondary target, i.e. the Bio-orthogonal Reactive Group (or groups) comprised in the Pre-targeting Probe. It will be understood that, to the extent that the secondary target is a cyclooctene, the secondary targeting moiety will be a tetrazine, and *vice versa.*

The Effector Moiety can, e.g., be a detectable label. A "detectable label" as used herein relates to the part of the Effector Probe which allows detection of the probe, e.g. when present in a cell, tissue or organism. One type of detectable label envisaged within the context of the present invention is a contrast providing agent. Different types of detectable labels are envisaged within the context of the present invention and are described hereinbelow.

Thus, according to a particular embodiment of the present invention, the pretargeting kits and methods of the present invention are used in imaging, especially medical imaging. In order to identify the primary target, use is made, as the Effector Probe, of an imaging probe comprising one or more detectable labels. Particular examples of detectable labels of the imaging probe are contrast -providing moieties used in traditional imaging systems such as MRI-imageable constructs, spin labels, optical labels, ultrasound-responsive constructs, X-ray-responsive moieties, radionuclides, (bio)luminescent and FRET-type dyes. Exemplary detectable labels envisaged within the context of the present invention include, and are not necessarily limited to, fluorescent molecules, e.g. autofluorescent molecules, molecules that fluoresce upon contact with a reagent, etc., radioactive labels; biotin, e.g., to be detected through binding of biotin by avidin; fluorescent tags, imaging constructs for MRI comprising paramagnetic metal, imaging reagents, e.g., those described in U.S. Pat. Nos. 4,741,900 and 5,326,856) and the like. The radionuclide used for imaging can be, for example, an isotope selected from the group consisting of ³H, ¹¹C, ¹³N, ¹⁵O, ¹⁸F, ¹⁹F, ⁵¹Cr, ⁵²Fe, ⁵²Mn, ⁵⁵Co, ⁶⁰Cu, ⁶¹Cu, ⁶²Zn, ⁶²Cu, ⁶³Zn, ⁶⁴Cu, ⁶⁶Ga, ⁶⁷Ga, ⁶⁸Ga, ⁷⁰As, ⁷¹As, ⁷²As, ⁷⁴As, ⁷⁵Se, ⁷⁵Br, ⁷⁶Br, ⁷⁷Br, ⁸⁰Br, ⁸²Br, ⁸²Rb, ⁸⁶Y, ⁸⁸Y, ⁸⁹Sr, ⁸⁹Zr, ⁹⁷Ru, ⁹⁹Tc, ¹¹⁰In, ¹¹¹In, ¹¹³In, ¹¹⁴In, ¹¹⁷Sn, ¹²⁰I, ¹²²Xe, ¹²³I, ¹²⁴I, ¹²⁵I, ¹⁶⁶Ho, ¹⁶⁷Tm, ¹⁶⁹Yb, ¹⁹³Pt, ¹⁹⁵Pt, ²⁰¹Tl, and ²⁰³Pb.

Other elements and isotopes, such as being used for therapy may also be applied for imaging in certain applications.

The MRI-imageable moiety can be a paramagnetic ion or a superparamagnetic particle. The paramagnetic ion can be an element selected from the group consisting of Gd, Fe, Mn, Cr, Co, Ni, Cu, Pr, Nd, Yb, Tb, Dy, Ho, Er, Sm, Eu, Ti, Pa, La, Sc, V, Mo, Ru, Ce, Dy, Tl. The ultrasound responsive moiety can comprise a microbubble, the shell of which consisting of a phospholipid, and/or (biodegradable) polymer, and/or human serum albumin. The microbubble can be filled with fluorinated gasses or liquids.

The X-ray-responsive moieties include but are not limited to iodine, barium, barium sulfate, gastrografin or can comprise a vesicle, liposome or polymer capsule filled with iodine compounds and/or barium sulfate.

Moreover, detectable labels envisaged within the context of the present invention also include peptides or polypeptides that can be detected by antibody binding, e.g., by binding of a detectable labeled antibody or by detection of bound antibody through a sandwich-type assay. In one embodiment the detectable labels are small size organic PET and SPECT labels, such as ¹⁸F, ¹¹C or ¹²³I. Due to their small size, organic PET or SPECT labels are ideally suited for monitoring intracellular events as they do not greatly affect the properties of the targeting device in general and its membrane transport in particular. An imaging probe comprising a PET label and either of the retro Diels-Alder active moieties as a secondary targeting moiety is lipophilic and able to passively diffuse in and out of cells until it finds its binding partner. Moreover, both components do not preclude crossing of the blood brain barrier and thus allow imaging of regions in the brain.

When the Effector Probe is intended to comprise a detectable label based on a metal, such as a lanthanide (e.g. Gd) for MRI contrast enhancement, such is preferably provided in the form of a chelate. In such a case the Effector Probe preferably comprises a structural moiety capable of forming a coordination complex with such a metal. A good example hereof are macrocylic lanthanide(III) chelates derived from 1,4,7,10-tetraazacyclododecane-1,4,7,10-tetraacetic acid (H₄dota), and 1,4,7,10-tetraazacyclododecane-α,α' ,α,"α'" -tetramethyl-1,4,7,10-tetraacetic acid (H₄dotma).

The Effector Moiety can also be a therapeutic moiety such as a pharmaceutically active compound. Examples of pharmaceutically active compounds are provided herein. A therapeutic probe can optionally also comprise a detectable label.

Thus, according to another embodiment, the pretargeting kits of the invention are used for targeted therapy. This is achieved by making use of an Effector Probe comprising a secondary targeting moiety and one or more pharmaceutically active agents (i.e. a drug or a radioactive isotope for radiation therapy). Suitable drugs for use in the context of targeted drug delivery are known in the art. Optionally, the therapeutic probe can also comprise a detectable label, such as one or more imaging agents. A radionuclide used for therapy can be an isotope selected from the group consisting of ²⁴Na, ³²P, ³³P, ⁴⁷Sc, ⁵⁹Fe, ⁶⁷Cu, ⁷⁶As, ⁷⁷As, ⁸⁰Br, ⁸²Br, ⁸⁹Sr, ⁹⁰Nb, ⁹⁰Y, ¹⁰³Ru, ¹⁰⁵Ph, ¹⁰⁹Pd, ¹¹¹Ag, ¹²¹Sn, ¹²⁷Te, ¹³¹I, ¹⁴⁰La, ¹⁴¹Ce, ¹⁴²Pr, ¹⁴³Pr, ¹⁴⁴Pr, ¹⁴⁹Pm, ¹⁴⁹Tb, ¹⁵¹Pm, ¹⁵³Sm, ¹⁵⁹Gd, ¹⁶¹Tb, ¹⁶⁵Dy, ¹⁶⁶Dy, ¹⁶⁶Ho, ¹⁶⁹Er, ¹⁷²Tm, ¹⁷⁵Yb, ¹⁷⁷Lu, ¹⁸⁶Re, ¹⁸⁸Re, ¹⁹⁸Au, ¹⁹⁹Au, ²¹¹At, ²¹¹Bi, ²¹²Bi, ²¹²Pb, ²¹³Bi, ²¹⁴Bi, ²²³Ra, and ²²⁵Ac.

Alternatively the drug in the therapeutic probe is selected from sensitizers for photodynamic therapy.

In the Effector Probe, the secondary targeting moiety, i.e. the second Bio-orthogonal Reactive Group and the effector moiety can be directly linked to each other. They can also be bound to each other via a linker, and furthermore they can both be linked to a secondary targeting scaffold. The linker can, independently, be selected from the same moieties, e.g. poly ethylene glycols, as discussed above. The secondary targeting scaffold can be e.g. a biopolymer such as a polypeptide.

The invention also relates to a pre-targeting method, using the retro Diels-Alder reaction. Herein a Pre-targeting Probe comprising a primary targeting moiety (e.g., an antibody, and antibody fragment, or a receptor binding peptide), functionalized with a a compound according to formula (7) mentioned above, or with a cyclooctene according to formula (1) above, respectively, is injected into a subject. After binding to the target (e.g. a primary or metastatic tumor lesion, an atherosclerotic plaque, an infracted area, an inflammation or infection site, etc.) and clearance from the circulation and from non-target tissues (e.g. blood, liver, spleen, kidney, etc.) an Effector Probe comprising a secondary targeting moiety, e.g. carrying an E-cyclooctene or tetrazine derivative, respectively (i.e. the reactive counterpart of the Bio-orthogonal Reactive Group present in the Pre-targeting Probe), and a drug or an imageable label, is injected. The Effector Probe binds to the primary targeting moiety and provides high contrast or selectively treats the disease site.

The invention also relates to the targeting of a general metabolic pathway, which is upregulated during a disease (like infection or cancer) such as DNA, protein, and membrane synthesis and carbohydrate uptake. Suitable probes comprise diene or dienophile labeled amino acids, sugars, nucleic acids and choline, analogous to the metabolic tracers currently used in the art,[¹¹C]-methionine, [¹⁸F]-fluorodeoxyglucose (FDG), deoxy-[¹⁸F]-fluorothymidine (FLT) and [¹¹C]-choline. Cells with a high metabolism or proliferation have a higher uptake of these building blocks. In this method, e.g. tetrazine- or E-cyclooctene derivatives enter these or other pathways and accumulate in and/or on cells. After sufficient build-up and clearance of free probe a detectably labeled or drug-carrying (cell permeable) tetrazine probe or E-cyclooctene probe (or probes carrying other dienes/dienophiles according to the invention) is sent in to bind the accumulated E-cyclooctene, respectively tetrazine metabolite. As an advantage over normal FDG (fluorine 18 fluorodeoxyglucose)-type imaging, ample time is available to allow high build up of the targeting moiety before radioactivity is sent in, thus increasing the target to non-target ratio. Alternatively, a metabolic pathway and/or metabolite that is specific for a disease can be targeted.

The invention also relates to the pre-targeting of intracellular targets. Due to their small size, organic PET labels (¹⁸F, ¹¹C) are ideally suited for monitoring intracellular events as they do not greatly affect the properties of the targeting device in general and its membrane transport in particular (contrary to the large and polar radiometal-chelate construct conjugates). Although the substituted tetrazine moiety and the E-cyclooctene used in the invention are not necessarily small, they are relatively nonpolar and can be used for intracellular imaging of proteins, mRNA, signaling pathways etc. The secondary (PET labeled) substituted tetrazine moiety or E-cyclooctene probe (i.e. the Effector Probe) is capable of passively diffusing in and out of cells until it finds its binding partner. These properties also allow the use of retro Diels-Alder reaction for pre-targeting in the brain, as both components do not preclude crossing of the blood brain barrier.

The invention also pertains to pretargeted signal amplification and/or polyvalency installation. At least one primary targeting device is conjugated to a dendrimer or polymer containing multiple tetrazine moieties. After receptor binding, an (one or more) cyclooctene or cyclooctyne conjugated to one or more contrast moieties for nuclear imaging (e.g., a radiometal chelate, a radiohalogen, etc.) or MRI (e.g., Gd chelates) is injected. The subsequent retro Diels-Alder reaction results in a high concentration of MRI contrast agent at the target tissue. Furthermore, the polyvalency at the target site will increase the reaction kinetics with the cyclooctene or cyclooctyne effector conjugate, affording an efficient target accumulation of for example MRI contrast agents. Naturally, the cyclooctene or cyclooctyne can also be used in the targeting device conjugate and the tetrazine (or other diene of the invention) conjugated to the reporter.

### Conjugation Route and Kits

The invention further pertains to the use of the retro Diels-Alder reaction as a route for the conjugation of imaging agents and drugs to targeting constructs such as peptides. The effector can contain organic PET labeled prosthetic groups, metal complexes for PET/SPECT/MRI and microbubbles for ultrasound imaging, but also α and β⁻ emitters for radiotherapy and, in general, a cytotoxic anticancer agent. The imaging/therapy agents can be functionalized with a pendant tetrazine moiety and the targeting group with a cyclooctene derivative, or vice versa.

The present route is especially advantageous for agents for nuclear imaging and radiotherapy: in view of the decay of the radionuclide it is beneficial to conduct the most time-consuming step (the actual targeting in the body of a subject) as a pre- targeting step. The selection, according to the invention, of the above-described very rapid retro Diels-Alder chemistry for the secondary targeting, allows for using a broad range of radionuclides, including shorter lived ones than with existing methods. Cyclooctene functionalized Effector Probes and suitable diene, e.g., tetrazine carrying Pre-targeting Probes can be coupled at extremely low concentrations *in vivo* without the need for sustained blood circulation of the effector moiety (such as the radionuclide). It will be understood that this equally holds for cyclooctene carrying Pre-targeting Probes combined with diene, particularly tetrazine, functionalized Effector Probes. Moreoever, the reactive groups are advantageously stable, and thus present a longer lived reactivity, without being too easily prone to side reactions.

It will be understood that the foregoing provides advantages such as minimizing the radiation dose to the patient. Also, it leads to allowing the usage of PET i.e. Positron Emission Tomography agents instead of SPECT i.e. Single Photon Emission Computerized Tomography agents.

The present invention is particularly suitable for use in multimodal imaging, optionally using different imaging agents to visualize the same target. Alternatively the imaging probe comprises at least 2 different labels to enable multimodal imaging.

The application of the [4+2] retro Diels-Alder chemistry in molecular imaging opens up pre-targeting to all types and sizes of targeting constructs. This allows intracellular and metabolic imaging to profit from the high target accumulation and low background, attainable through pre-targeting build-up. Likewise, pre-targeted signal amplification schemes, e.g. polytetrazine and/or polyalkene dendrimers or liposomes, become available for smaller and more diverse targeting devices.

As the reaction partners are abiotic and bio-orthogonal, pre-targeting using the [4+2] retro Diels-Alder reaction as described above, is not hampered by endogenous competition and metabolism/decomposition, and affords a stable covalent bond. Choosing a target metabolic pathway, and the corresponding tetrazine-metabolite derivative by virtue of its high flux in, for example, tumor cells compared to normal cells, affords the installation of a high density of artificial tetrazine receptors or other chemical handles in cells or on the surfaces of target cells, circumventing the use of endogenous cell surface receptors which can sometimes be at low levels.

Further particular embodiments of the present invention relate to kits comprising a metabolic precursor and an imaging probe, more particularly an imaging probe comprising a detectable label, which is a contrast agent used in traditional imaging systems. Such a detectable label can be but is not limited to a label selected from the group consisting of MRI-imageable constructs, spin labels, optical labels, ultrasound-responsive agents, X-ray-responsive agents, radionuclides, and FRET -type dyes. In a particular embodiment of the present invention, use is made of reporter probes. Such a reporter probe can be the substrate of an enzyme, more particularly an enzyme which is not endogenous to the cell, but has been introduced by way of gene therapy or infection with a foreign agent. Non-endogenous as referring to a gene in a cell or tissue herein is used to indicate that the gene is not naturally present and/or expressed in that cell or tissue. Alternatively, such a reporter probe is a molecule which is introduced into the cell by way of a receptor or a pump, which can be endogenous or introduced into the cell by way of gene therapy or infection with a foreign agent. Alternatively, the reporter probe is a molecule which reacts to certain (changing) conditions within a cell or tissue environment.

The invention also includes agents for use in the kits described above. One such agent is a pretargeting agent comprising a primary targeting moiety and a bio-orthogonal reactive group, wherein the bio-orthogonal reactive group is a reaction partner for a [4+2] retro Diels-Alder reaction. Particular reaction partners are described hereinbefore, i.e. an electron-deficient tetrazine as discussed above, or a cyclooctene (preferably an E-cyclooctene). The invention also relates to the use of these agents in targeted medical imaging or targeted therapy, and to these agents for use in such a method. Particularly, the invention relates to these use of these agents in a pretargeting method, and to these agents for use in such a method. Another such agent is an imaging probe comprising a detectable label and a bio-orthogonal reactive group, wherein the bio-orthogonal reactive group is a reaction partner for a [4+2] retro Diels-Alder reaction.

The invention also relates to an imaging probe comprising a detectable label and a bio-orthogonal reactive group, wherein the bio-orthogonal reactive group is a reaction partner for a [4+2] retro Diels-Alder reaction. The invention further relates to a therapeutic probe comprising a pharmaceutically active compound and a bio-orthogonal reactive group, wherein the bio-orthogonal reactive group is a reaction partner for a [4+2] retro Diels-Alder reaction.

Part of the invention is also a pretargeting method comprising administering a pretargeting agent as described above to a subject and allowing the agent to circulate in the subject's system for a period of time effective to achieve binding of the primary targeting moiety to a primary target, followed by clearing non-bound agent from the body. A typical time period for this is 12 to 96 hours, particularly around 48 hours.

Further, the invention provides an imaging method comprising conducting a pretargeting method as described above, followed by the administration of an imaging probe also according to the invention, wherein the bio-orthogonal reactive groups in the pretargeting agent and in the imaging probe together form the reactive partners for the [4+2] retro Diels-Alder reaction. Similarly, the invention provides a method of targeted medical treatment in a subject, comprising conducting a pretargeting method as described above, followed by the administration of a therapeutic probe also according to the invention, wherein the bio-orthogonal reactive groups in the pretargeting agent and in the imaging probe together form the reactive partners for the [4+2] retro Diels-Alder reaction.

The invention also pertains to the aforementioned pretargeting agents for use in an imaging method as described above.

In summary, on the basis of retro Diels-Alder chemistry, bio-orthogonal pretargeted molecular imaging and therapy serves to bring great advantages to patients. On one side, it serves to afford the acquisition of superior images of target tissues such as cancer and cardiovascular lesions. On the other hand, the intrinsic side effects deriving from the administration of radioactive compounds and, in general, potentially toxic drugs can be greatly diminished while increasing the effective dose that reaches a diseased tissue. Furthermore, it will greatly expand the collection of traceable molecular events that underlie disease. In particular, this technology can give access to target tissues far from blood vessels and will facilitate imaging of the information-rich intracellular environment.

The invention will be illustrated with reference to the following, non-limiting Examples and the accompanying non-limiting Figs..

### Example 1

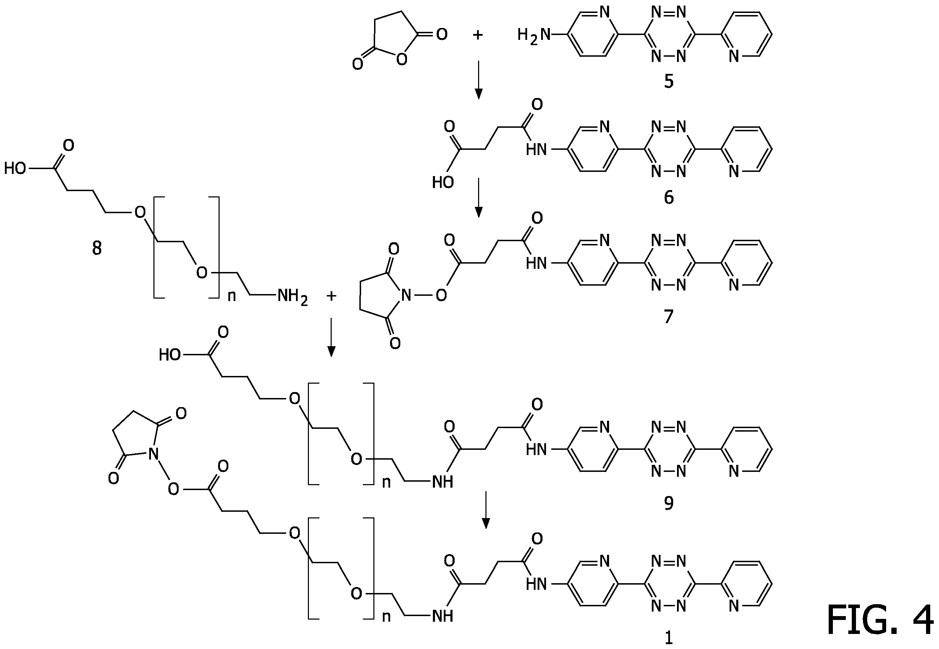

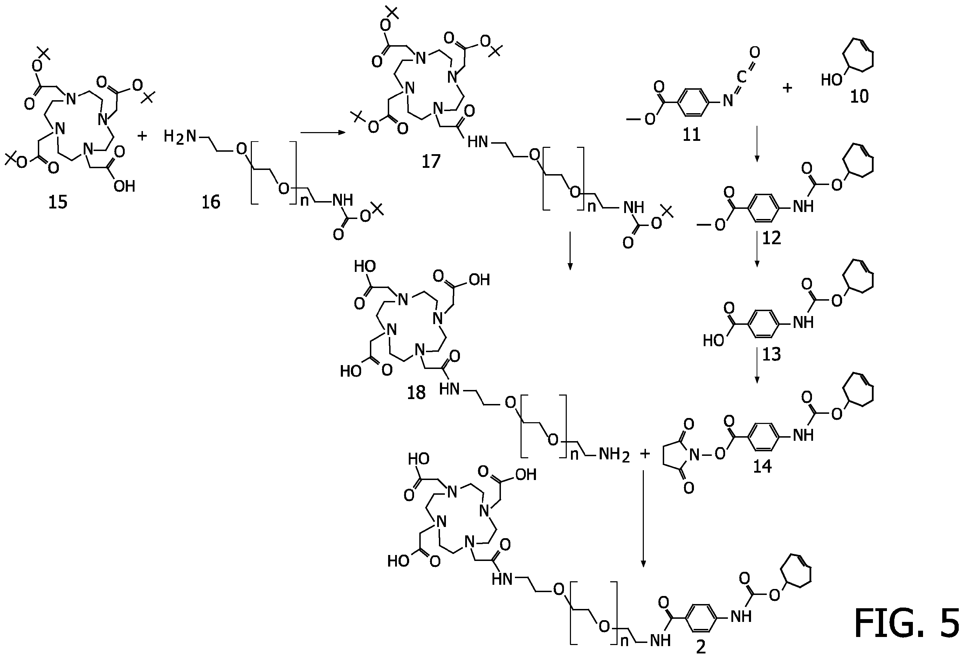

As an example to link the tetrazine derived moiety to an antibody as outlined in Fig. 3a, a molecule **1** (see Fig. 4) is prepared. An example of a corresponding probe **2**, derived from E-cyclooctene, is presented in Fig. 5. Both molecules contain PEG chains. Molecule **1** comprises an N-hydroxysuccimidyl moiety, that is used to couple the molecule with amino groups present in the antibody. The DOTA derived moiety in **2** can be used to carry a rare earth metal ion such as Gd for MR imaging or Lu-177 for nuclear imaging and therapy (SPECT).

The synthesis of **1** is outlined in Fig. 4. The starting tetrazine derived molecule **5** is made according to Blackman et al. (Blackman, ML; Royzen, M; Fox, JM, Journal of The American Chemical Society, 2008, 130 (41), 13518-19). It is converted to the acid **6** by reaction with succinic anhydride followed by formation of its N-hydroxysuccimidyl ester **7.** This N-hydroxysuccimidyl ester is used to form acid **9** by reaction with the commercially available (IRIS biochem) PEG derivative **8** that in its turn is converted into its N-hydroxysuccimidyl ester **1.**

The synthesis of **2** is outlined in fig. 5. (E)-cyclooct-4-enol (**10**) is prepared according to Yap et al. (Yap, GPA; Royzen, M; Fox, JM, Journal of The American Chemical Society, 2008, 130 (12), 3760 -61). With the aid of the commercially available (Aldrich) isocyanate derivative **11** it is converted into ester **12**, followed by saponification to acid **13.** N-hydroxysuccimidyl ester **14** formed out of **13** is made to react with the DOTA and PEG derived amine **18** to form the final product **2.** DOTA derivative **18** is prepared after deprotection of the **17** that in turn is prepared from the DOTA derivative **15** and PEG derivative **16**, both available commercially (from Macrocyclics and IRIS biotech, respectively).

### Example 2

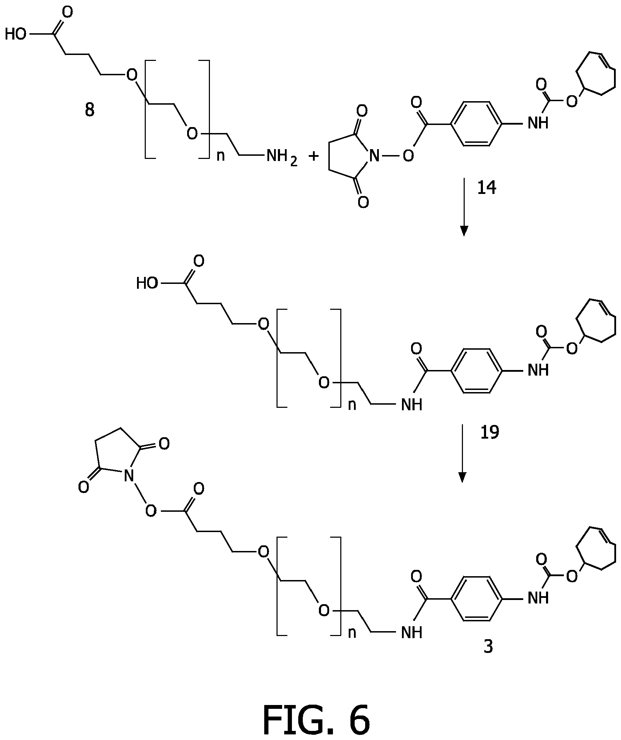

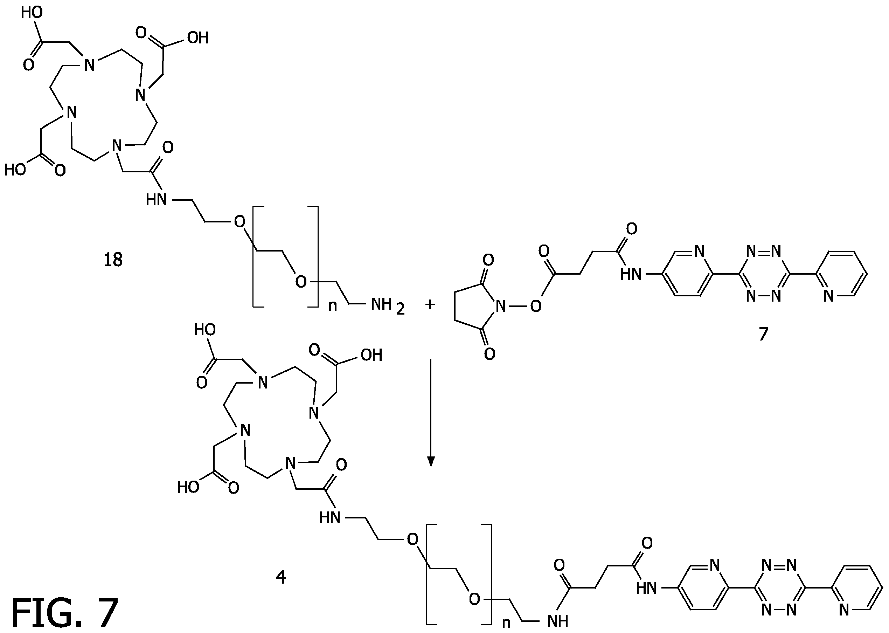

As compared to Example 1, this example illustrates the inverse pair of molecules namely, 1) the E-cyclooctene derivative **3** meant to form the pretargeting moiety after conjugating to the antibody and, 2) the tetrazine / DOTA derived probe **4** that can serve as the Effector Probe as outlined in Fig. 3b, are shown in Figs. 6 and 7, respectively.

E-cyclooctene derivative **3** is formed by reaction of the commercially available (IRIS biochem) PEG derivative **8** with N-hydroxysuccimidyl ester **14** (see Fig. 5) to form acid **19,** followed by formation of the N-hydroxysuccimidyl derivative out of this acid.

The synthesis of the tetrazine / DOTA derived probe **4** is outlined in Fig. 7. This probe is made by reaction of the DOTA and PEG derived amine **18** (see Fig. 5) with N-hydroxysuccimidyl ester **7** (see Fig. 4).

### Example 3: In vivo imaging

All reagents and solvents were obtained from commercial sources (Sigma-Aldrich, Acros, ABCR, Invitrogen, and Merck for reagents, Biosolve, Merck and Cambridge Isotope Laboratories for normal and deuterated solvents) and used without further purification unless stated otherwise. 1-Amino-3,6,9,12,15,18,21,24,27,30,33,36-dodecaoxanonatriacontan-39-oic acid (S11) and tert-butyl (35-amino-3,6,9,12,15,18,21,24,27,30,33-undecaoxapentatriacontyl)carbamate (S3) were obtained from Polypure (Norway) and Iris Biotech (Germany), respectively. 2,2',2"-(10-(2-((2,5-Dioxopyrrolidin-1-yl)oxy)-2-oxoethyl)-1,4,7,10-tetraazacyclododecane-1,4,7-triyl)triacetic acid (S6) as a salt with HPF₆ and approximately 3 eq. of trifluoroacetic acid (TFA) was obtained from Macrocyclics (USA). Rituximab solutions (MabThera®) were purchased from Roche (Switzerland). [¹¹¹In]Indium chloride and sodium [¹²⁵I]iodide solutions were purchased from PerkinElmer (USA). Water was distilled and deionized (18 mΩcm⁻¹) by means of a milli-Q water filtration system (Millipore, USA). The labeling buffers were treated with Chelex-100 resin (BioRad Laboratories, USA) overnight, then filtered through 0.22 µm and stored at 4°C. Iodogen iodination tubes, kits for bicinchoninic acid (BCA) assay and gelcode blue protein staining solutions were purchased from Pierce Protein Research (Thermo Fisher Scientific, USA). Tablets to prepare phosphate buffered saline (PBS) pH 7.4 were acquired from Calbiochem (Merck, Germany). Amicon Ultra-4 and Ultra-15 centrifugal filter units (50 kDa MW cut-off) were purchased from Millipore. Mouse serum was purchased from Innovative Research (USA).

NMR spectra were recorded in deuterated chloroform (CDCl₃) or hexadeuterated dimethylsulfoxide (DMSO-D₆), using a Bruker DPX300 spectrometer or a Bruker Avance600 spectrometer (Bruker BioSpin, The Netherlands). ¹³C-NMR multiplicities (q=quaternary, t=tertiary, s=secondary and p=primary) were distinguished using a DEPT pulse sequence. High-resolution ESI mass spectra (HRMS) were recorded on an Agilent ESI-TOF mass spectrometer (Agilent Technologies, USA), measuring in the positive ion mode. Preparative column chromatography was performed on a Combiflash Companion apparatus (Teledyne Isco, USA) using silica columns (SiliCycle, Canada). Preparative HPLC was performed using an Agilent 1200 apparatus, equipped with a C18 Zorbax column (21.2×150mm, 5µm particles) applying a gradient of water and acetonitrile (ACN) containing 0.1% TFA. Analytical radio-HPLC was carried out on an Agilent 1100 system equipped with a Gabi radioactive detector (Raytest, Germany). The samples were loaded on an Agilent Eclipse XDB-C18 column (4.6×150mm, 5µm particles), which was eluted at 1 mL/min with a linear gradient of ACN in water containing 0.1% TFA (2 min at 10% ACN followed by an increase to 45% ACN in 11 min). The UV wavelength was preset at 254 nm. Size exclusion (SEC) HPLC was carried out on an Agilent 1200 system equipped with a Gabi radioactive detector. The samples were loaded on a BioSep-SEC-S 2000 column (300×7.8mm, 5µm particles, Phenomenex, USA) and eluted with 20 mM phosphate, 150 mM NaCl, pH 6.8, at 1 mL/min. The UV wavelength was preset at 260 and 280 nm.

The ¹¹¹In-labeling yields were determined by radio-TLC, using ITLC-SG strips (Pall, USA) eluted with 200 mM ethylenediaminetetraacetic acid in 0.9% aq. NaCl and imaged on a phosphor imager (FLA-7000, Fujifilm, Japan). In these conditions, free ¹¹¹In migrates with R_{f} = 0.9, while ¹¹¹In-tetrazine remains at the origin. The ¹²⁵I-labeling yields were also determined with radio-TLC, using ITLC-SG strips eluted with a 20 mM citric acid solution (pH 5.2) and imaged on a phosphor imager. In these conditions, free ¹²⁵I migrates with R_{f} = 0.9, while ¹²⁵I-mAbs remain at the origin.

Isoelectric focusing (IEF) analysis and SDS-PAGE were performed on a Phastgel system using IEF-3-9 gels and 7.5% PAGE homogeneous gels (GE Healthcare Life Sciences, USA), respectively. The IEF calibration solution (broad PI, pH 3-10) was purchased from GE Healthcare and the protein MW standard solution (Precision Plus dual color standard) was purchased from BioRad. Upon electrophoresis, the gels were stained for 2 h with gelcode blue, destained overnight in water and then digitized with a conventional flat bed scanner.

The concentration of CC49 and rituximab solutions was determined with a NanoDrop 1000 spectrophotometer (absorbance at 280 nm; Thermo Fisher Scientific, The Netherlands) or with a BCA test.

### Synthesis of DOTA tetrazine (S7)

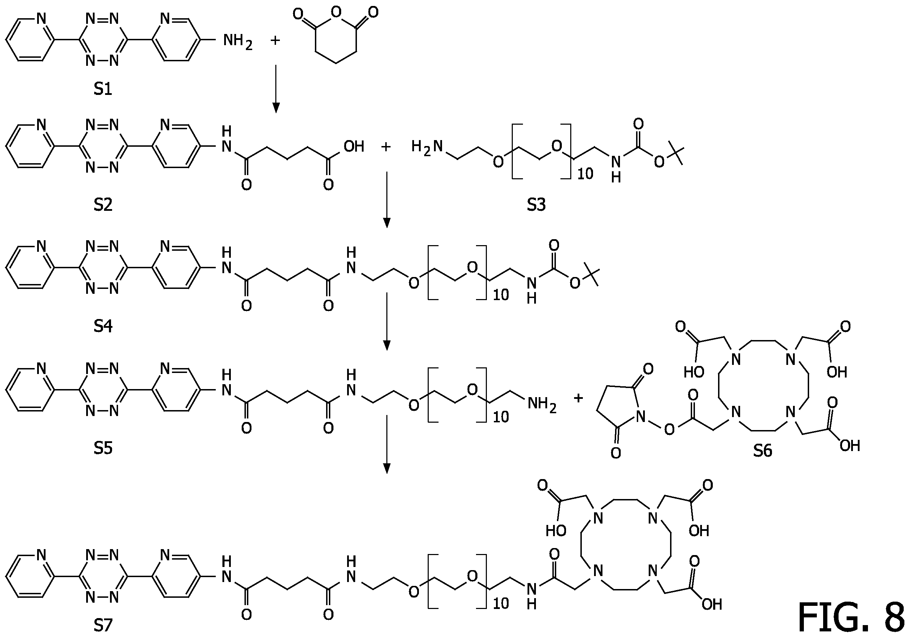

Synthesis overview of 2,2',2"-(10-(2,40,44-trioxo-44-((6-(6-(pyridin-2-yl)-1,2,4,5-tetrazin-3-yl)pyridin-3-yl)amino)-6,9,12,15,18,21,24,27,30,33,36-undecaoxa-3,39-diazatetratetracontyl)-1,4,7,10-tetraazacyclododecane-1,4,7-triyl)triacetic acid (S7) is shown in figure 8.

### 5-Oxo-5-(6-(6-(pyridin-2-yl)-1,2,4,5-tetrazin-3-yl)pyridin-3-ylamino)pentanoic acid (S2).

6-(6-(Pyridin-2-yl)-1,2,4,5-tetrazin-3-yl)pyridin-3-amine (**S1**) was synthesized according to a literature procedure (M. L. Blackman, M. Royzen, J. M. Fox, J. Am. Chem. Soc. 130, 13518 (2008).). A mixture of **S1** (103 mg, 0.410 mmol) and glutaric anhydride (234 mg, 2.05 mmol) in tetrahydrofuran (12 mL) was heated at 70°C for 18 h in a sealed flask. After cooling, the precipitate was washed with dichloromethane (DCM) (2×12 mL) and ethyl acetate (12 mL) to yield **S2** as a purple solid (62 mg, 42%). ¹H NMR (DMSO-D₆, 300 MHz, δ): 12.13 (s, 1H), 10.58 (s, 1H), 9.05 (d, J = 2.3 Hz, 1H), 8.94 (d, J = 4.2 Hz, 1H), 8.62 (d, J = 8.8 Hz, 1H), 8.60 (d, J = 8.8 Hz, 1H), 8.43 (dd, J₁ = 2.3 Hz, J₂ = 8.8 Hz, 1H), 8.16 (td, J₁ = 7.8 Hz, J₂ = 1.7 Hz, 1H), 7.73 (ddd, J₁ = 1.1 Hz, J₂ = 4.4 Hz, J₃ = 7.4 Hz), 2.50 (t, J = 7.3 Hz, 2H), 2.33 (t, J = 7.3 Hz, 2H), 1.86 (q, J = 7.3 Hz, 2H). ¹³C NMR (DMSO-D₆, 75 MHz, δ): 174.1 (q), 172.0 (q), 163.0 (q), 162.7 (q), 150.6 (t), 150.2 (q), 143.8 (q), 141.3 (t), 138.4 (q), 137.7 (t), 126.5 (t), 126.1 (t), 124.8 (t), 124.1 (t), 35.4 (s), 32.9 (s), 20.2 (s). HRMS (ESI, m/z): Calcd for C₁₇H₁₆N₇O⁺ ([M+H]⁺): 366.1314; Found: 366.1313.

### Tert-butyl (37,41-dioxo-41-((6-(6-(pyridin-2-yl)-1,2,4,5-tetrazin-3-yl)pyridin-3-yl)amino)-3,6,9,12,15,18, 21,24,27,30,33-undecaoxa-36-azahentetracontyl)carbamate (S4).

N,N-Diisopropylethylamine (95 µL, 0.41 mmol) was added to a stirred mixture of **S2** (15 mg, 0.041 mmol), **S3** (29 mg, 0.045 mmol), and (benzotriazol-1-yloxy)tris(dimethylamino)phosphonium hexafluorophosphate (19 mg, 0.043 mmol) in dimethylformamide (DMF) (1 mL). After stirring for 16 h at room temperature (RT), the mixture was evaporated and the product was purified by column chromatography on silica using a gradient of methanol in DCM (0-10%) giving **S4** as a purple solid (30 mg, 74%). ¹H NMR (CDCl₃, 300 MHz, δ): 9.07 (d, J = 2.2 Hz, 1H), 8.97 (d, J = 4.6 Hz, 1H), 8.73 (d, J = 8.8 Hz, 1H), 8.72 (d, J = 8.8 Hz, 1H), 8.63 (dd, J₁ = 2.3 Hz, J₂ = 8.8 Hz, 1H), 8.03 (td, J₁ = 7.8 Hz, J₂ = 1.7 Hz, 1H), 7.59 (ddd, J₁ = 1.1 Hz, J₂ = 4.6 Hz, J₃ = 7.4 Hz), 7.01 (s, 1H), 5.14 (s, 1H), 3.9-3.2 (broad s, 48H), 2.60 (t, J = 7.1 Hz, 2H), 2.37 (t, J = 7.1 Hz, 2H), 2.09 (q, J = 7.1 Hz, 2H), 1.43 (s, 9H). ¹³C NMR (CDCl₃, 75 MHz, δ): 173.8 (q), 173.1 (q), 163.8 (q), 163.7 (q), 151.3 (t), 150.5 (q), 144.0 (q), 142.7 (t), 139.2 (q), 137.9 (t), 127.0 (t), 126.9 (t), 125.5 (t), 124.7 (t), 79.5 (q), 70.5 (s), 70.1 (s), 40.7 (s), 39.7 (s), 36.5 (s), 35.5 (s), 28.8 (p), 21.9 (s). HRMS (ESI, m/z): Calcd for C₄₆H₇₄N₉O₁₅⁺ ([M+H]⁺): 992.5304; Found: 992.5301.

### 2,2',2"-(10-(2,40,44-Trioxo-44-((6-(6-(pyridine-2-yl)-1,2,4,5-tetrazin-3-yl)pyridine-3-yl)amino)-6,9,12,15,18,21,24,27,30,33,36-undecaoxa-3,39-diazatetratetracontyl)-1,4,7,10-tetraazacyclododecane-1,4,7-triyl)triacetic acid (S7).

Product **S4** (30 mg, 0.030 mmol) was stirred for 30 min at RT in a mixture of TFA (1.5 mL) and DCM (5 mL). After evaporation, the residue (mainly **S5**) was dissolved in DMF (5 mL), **S6** (27 mg, 0.028 mmol) and triethylamine (40 µL, 0.27 mmol) were added and the mixture was stirred for 2 h at RT. After evaporation, the crude material was dissolved in DMSO and purified by preparative HPLC. After lyophilisation, **S7** (24 mg, 69%) was obtained as a purple TFA salt, as confirmed by ¹³C-NMR analysis (δ=158.7ppm, q, J=32.3Hz and δ=117.6ppm, q, J=298.2Hz). ¹H NMR (DMSO-D₆, 600 MHz, δ): 9.22 (d, J = 2.4 Hz, 1H), 9.10 (dd, J₁ = 4.7 Hz, J₂ = 1.1 Hz, 1H), 8.78 (d, J = 8.7 Hz, 1H), 8.75 (d, J = 7.8 Hz, 1H), 8.59 (dd, J₁ = 2.4 Hz, J₂ = 8.7 Hz, 1H), 8.32 (td, J₁ = 7.8 Hz, J₂ = 1.7 Hz, 1H), 8.08 (ddd, J₁ = 1.1 Hz, J₂ = 4.7 Hz, J₃ = 7.8 Hz), 3.9-3.1 (m, 80H), 2.60 (t, J = 7.3 Hz, 2H), 2.34 (t, J = 7.3 Hz, 2H), 2.02 (q, J = 7.3 Hz, 2H). ¹³C NMR (DMSO-D₆, 150 MHz, δ): 172.8 (q), 172.5 (q), 163.8 (q), 163.5 (q), 151.3 (t), 150.9 (q), 144.5 (q), 142.0 (t), 139.3 (q), 138.5 (t), 127.3 (t), 126.9 (t), 125.6 (t), 124.9 (t), 70.5 (s), 70.3 (s), 69.9 (s), 69.5 (s), 39.2 (s), 36.4 (s), 35.2 (s), 21.7 (s). HRMS (ESI, m/z): Calcd for C₅₇H₉₂N₁₃O₂₀⁺ ([M+H]⁺): 1278.6582; Found: 1278.6557.

### Synthesis of TCO-NHS, S13

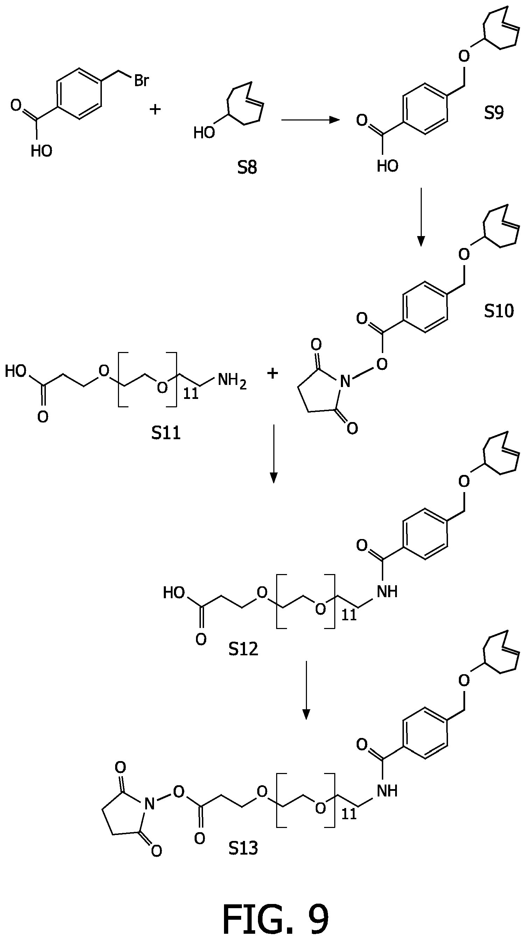

Synthesis of (E)-2,5-dioxopyrrolidin-1-yl 1-(4-((cyclooct-4-en-1-yloxy)methyl)phenyl)-1-oxo-5,8,11,14,17,20,23,26,29,32,35,38-dodecaoxa-2-azahentetracontan-41-oate (S13) is shown in figure 9.

### (E)-2,5-Dioxopyrrolidin-1-yl 4-((cyclooct-4-enyloxy)methyl)benzoate (S10).

(E)-Cyclooct-4-enol (**S8**, major isomer containing approximately 13% of the Z-isomer) was synthesized according to a literature procedure (M. Royzen, G. P. A. Yap, J. M. Fox, J. Am. Chem. Soc. 130, 3760 (2008)). A 60% sodium hydride dispersion (1.8 g, 45 mmol) was added to an ice-bath-cooled solution of **S8** (1.70 g, 13.5 mmol) in 60 mL DMF. After stirring for 4 h at RT, 4-bromomethylbenzoic acid (3.85 g, 17.9 mmol) was added in portions and the suspension was stirred overnight at RT. The mixture was poured into water (100 mL), t-butyl methyl ether (100 mL) was added followed by 37% hydrochloric acid (5 mL). After separation, the aqueous layer was extracted with t-butyl methyl ether (2×100 mL). The combined organic layers were washed with water (25 mL), dried over MgSO₄ and evaporated. The residue was passed through a thin silica layer with 4:1 hexane/ethyl acetate. The residue obtained after evaporation was dissolved in heptane (50 mL) at 70°C and then cooled, affording **S9.** The product was dissolved in DCM (40 mL), N-hydroxysuccinimide (0.57 g, 4.9 mmol) was added, the mixture was cooled in an ice-bath, followed by addition of N,N'-dicyclohexylcarbodiimide (1.03 g, 4.99 mmol). After 30 min the ice-bath was removed and the reaction mixture was stirred at RT for 18 h. After filtration and evaporation, the residue was purified by column chromatography on silica using a gradient of ethyl acetate in heptane (0-15%). Next, the residue was dissolved in t-butyl methyl ether (20 mL) and poured into heptane (50 mL), yielding **S10** (1.42 g, 29%) as a white solid. ¹H NMR (CDCl₃, 300 MHz, δ): 8.10 (d, J = 8.5 Hz, 2H), 7.45 (d, J = 8.5 Hz, 2H), 5.60 (m, 1H), 5.34 (m, 1H), 4.54 (d, J = 13.4 Hz, 1H), 4.47 (d, J = 13.4 Hz, 1H), 3.09 (m, 1H), 2.91 (s, 4H), 2.43-1.40 (m, 10H). ¹³C NMR (CDCl₃, 75 MHz, δ): 169.0 (q), 161.5 (q), 146.7 (q), 135.1 (t), 132.1 (t), 130.4 (t), 127.0 (t), 123.7 (q), 85.3 (t), 68.0 (s), 40.5 (s), 37.7 (s), 34.2 (s), 32.7 (s), 31.4 (s), 25.4 (s). HRMS (ESI, m/z): Calcd for C₂₀H₂₃NO₅Na⁺ ([M+Na]⁺): 380.1474; Found: 380.1472.

### (E)-2,5-Dioxopyrrolidin-1-yl 1-(4-((cyclooct-4-en-1-yloxy)methyl)phenyl)-1-oxo-5,8,11,14,17,20,23,26, 29,32,35,38-dodecaoxa-2-azahentetracontan-41-oate (S13).

A solution of **S10** (100 mg, 0.280 mmol) in DCM (2 mL) was added dropwise to a solution of **S11** (175 mg, 0.283 mmol) and triethylamine (290 µL, 2.08 mmol) in DCM (2 mL) stirred in an ice-bath. The reaction mixture was stirred at RT for 16 h. The crude intermediate **S12** obtained after evaporation was dissolved in DCM (5 mL) and cooled in an ice bath. Bis(2,5-dioxopyrrolidin-1-yl) carbonate (170 mg, 0.664 mmol) and pyridine (28 µL, 0.35 mmol) were added and the reaction mixture was stirred at RT for 3 h. The mixture was filtered and evaporated and the product was purified by column chromatography on silica using a gradient of methanol in DCM (5-10%) affording **S13** as a viscous oil (119 mg, 39%). ¹H NMR (CDCl₃, 600 MHz, δ): 7.79 (d, J = 8.2 Hz, 2H), 7.36 (d, J = 8.2 Hz, 2H), 7.00 (s, 1H), 5.59 (m, 1H), 5.33 (m, 1H), 4.49 (d, J = 12.5 Hz, 1H), 4.42 (d, J = 12.5 Hz, 1H), 3.85 (t, J = 6.5 Hz, 2H), 3.8-3.5 (m, 48H), 3.09 (m, 1H), 2.90 (t, J = 6.5 Hz, 2H), 2.85 (s, 4H), 2.43-1.40 (m, 10H). ¹³C NMR (CDCl₃, 150 MHz, δ): 167.0 (q), 165.4 (q), 164.8 (q), 140.8 (q), 133.5 (t), 131.7 (q), 130.4 (t), 125.3 (t), 83.4 (t), 68.7 (s), 68.4 (s), 68.0 (s), 67.6 (s), 68.4 (s), 63.9 (s), 38.9 (s), 37.9 (s), 36.1 (s), 32.6 (s), 31.1 (s), 30.3 (s), 29.8 (s), 23.7 (s). HRMS (ESI, m/z): Calcd for C₄₇H₇₆N₂O₁₈Na⁺ ([M+Na]⁺): 957.5171; Found: 957.5174.

### Antibody production.

CC49 was produced from the CC49 hybridoma cell line acquired from the American Type Culture Collection (ATCC, USA). Hybridoma cells were grown in a CELLine CL 1000 bioreactor (Integra Biosciences AG, Switzerland) in serum-free hybridoma medium (H-SFM, Gibco, USA) supplemented with penicillin (10 U/ml) and streptomycin (10 µg/ml). Every two weeks the cell supernatant was collected and the CC49 was purified by protein G affinity chromatography using a MabTrap kit (GE Healthcare Biosciences, USA) according to the manufacturer's instructions. The purified CC49 was washed with PBS using an Amicon Ultra-15 centrifugal unit. This procedure afforded a CC49 solution containing a single species of approximately 150 kDa, as confirmed by SDS-PAGE and SEC-HPLC analysis.

### Antibody modification.

Typically, 2 mg CC49 (5 mg/mL solution in PBS) was modified with 10 molar eq. of TCO-NHS (S13, 127.6 µg in 12.8 µl DMSO) in a total volume of 500 µL PBS. The pH was adjusted to 9 with 2M sodium carbonate buffer. The reactions were carried out under agitation for 30 min at RT in the dark. Subsequently, the TCO-modified mAb was extensively washed with PBS using an Amicon Ultra-15 centrifugal device. This procedure afforded an average 7.4 TCO groups per antibody, as determined with a tetrazine titration (vide infra). The MW increase resulting from the TCO conjugation was not detectable by SDS-PAGE. As expected, the mAb modification resulted in a decrease in isoelectric point (from 6.5-6.8 to ca. 5.8) as observed by IEF analysis. The same procedure was used to produce TCO-modified rituximab (Rtx-TCO) for control in vivo experiments, with similar results.

### Antibody radiolabeling.

Native or TCO-modified CC49 (200 µg) in PBS (500 µL) was transferred to an iodination tube, which was pre-rinsed with 1 mL PBS. Sodium [¹²⁵I]iodide (10-15 MBq) was added, the solution was incubated for 5 min at RT under gentle agitation after which it was transferred into an Amicon Ultra-4 unit. The iodination tube was rinsed twice with 500 µL PBS and the washings were pooled with the labeling mixture. The ¹²⁵I-labeled mAb was washed extensively with PBS and subsequently recovered from the Amicon. With this procedure, 88.3±9.3 radioiodination yield (n=5) was obtained for native CC49 and 30.4±4.3% (n=6) for TCO-modified CC49. After Amicon purification, the radiochemical purity of both radiolabeled mAbs was greater than 98% as confirmed by radio-TLC and SDS-PAGE analysis. The same procedure was used to radioiodinate Rtx-TCO for control in vivo experiments, with similar results. For animal experiments, the specific activity (SA) of the purified ¹²⁵I-labeled mAbs was adjusted to 2 kBq/µg by adding an appropriate amount of the corresponding unlabeled mAb.

### Tetrazine radiolabeling.

The DOTA-modified tetrazine (probe S7) was dissolved (1 mg/mL) in 0.2M ammonium acetate pH 7.0 and stored at -80°C before use. One S7 aliquot was combined with a suitable amount of [¹¹¹In]indium chloride and incubated for 10 min at 37°C under gentle agitation. Then, 5 µL 10 mM diethylenetriaminepeantaacetic acid was added and the solution was incubated for an additional 5 min. Typically, a quantitative labeling yield and a radiochemical purity greater than 98% were obtained with this method, as confirmed by radio-HPLC and radio-TLC. For animal experiments, the ¹¹¹In-tetrazine solution was diluted with sterile saline. The SA of the ¹¹¹In-tetrazine solution used for in vitro experiments and for biodistribution studies was typically 50-100 kBq/µg S7; the SA for imaging experiments was 1-2 MBq/µg S7.

### In vitro reactivity.

The reactivity of ¹¹¹In-tetrazine towards CC49-TCO was tested in PBS (n=3), 50% mouse serum (n=3) and rat blood with heparin. Typically, 50 µg TCO-modified mAb was incubated with 0.43, 4.30 and 6.40 µg ¹¹¹In-labeled **S7** (1, 10 and 15 molar eq. with respect to the mAb) at 37°C in 100 µL total volume. Mixtures of unmodified CC49 and 15 eq. ¹¹¹In-labeled **S7** were used to evaluate non-specific binding. After 10 min, an aliquot of each mixture was analyzed by SDS-PAGE and phosphor imager. We observed a fast reaction between both components *in vitro* in semi-equimolar conditions and at low concentration (3.3 µM) within 10 min in PBS, serum and blood. The same procedure was used to evaluate the reactivity of ¹¹¹In-tetrazine towards Rtx-TCO. The reaction between ¹¹¹In-tetrazine and CC49-TCO in all three media was complete within 10 min (Table 1).

No appreciable binding between the labeled tetrazine and unmodified CC49 or other media components was detected, demonstrating the absence of non-specific interactions.

**Table 1:Reaction yields (%) between ¹¹¹In-tetrazine (eq. with respect to mAb) and CC49-TCO after 10 min incubation (parentheses: # tetrazine probes bound per mAb).**

| | PBS | Serum | Blood |
|---|---|---|---|
| 1 eq. | 86.5±1.3 (0.9±0.0) | 88.3±1.8 (0.9±0.0) | 87.0 (0.9) |
| 10 eq. | 74.7±6.9 (7.5±0.7) | 72.0±1.3 (7.2±0.1) | 72.6 (7.3) |
| 15 eq. | 50.0±1.5 (7.5±0.2) | 48.5±0.4 (7.3±0.1) | 50.0 (7.5) |
| Control (15 eq.) | 0.3±0.3 | 0.2±0.2 | 0.0 |

### In Vivo Studies

All animal experiments were performed according to the principles of laboratory animal care (NIH publication 85-23, revised 1985) and the Dutch national law "Wet op de Dierproeven" (Stb 1985, 336). The human colon cancer cell line LS174T was obtained from the ATCC and maintained in Eagle's minimal essential medium (Sigma) supplemented with 10% heat inactivated fetal calf serum (Gibco), penicillin (100 U/mL), streptomycin (100 µg/mL) and 2 mM Glutamax. Nude female Balb/C mice (20-25 g body weight, Charles River Laboratories, The Netherlands) were inoculated subcutaneously with 5×10⁶ cells in 100 µL sterile PBS. The tumor weight at the time of the imaging and biodistribution experiments was 0.38±0.28 g. Animals with different tumor sizes were randomly assigned to different experimental groups.

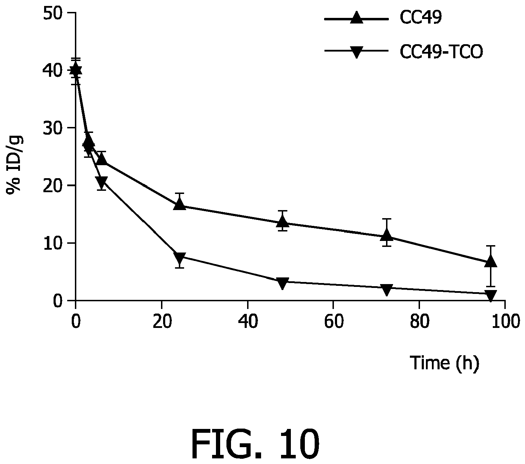

Tumor bearing mice (n=4) were injected intravenously with ¹²⁵I-CC49 or ¹²⁵I-CC49-TCO (100 µg/100 µL per mouse, ca. 0.2 MBq). At selected time points (5 min, 3 and 6 h, 1, 2 and 3 days) blood samples were withdrawn from the vena saphena. Four days after the injection, the mice were anesthetized with isoflurane and blood was withdrawn by heart puncture. The blood samples were weighed and diluted to 1 mL with PBS. The sample radioactivity was measured in a γ-counter (Wizard 1480, PerkinElmer) along with standards to determine the percent injected dose per gram (%ID/g). The half-life of the radiolabeled mAbs in blood was calculated from the area under the curves (AUC, GraphPad Prism v. 5.01) in Fig. 10 using T_{1/2} = Ln2×AUC/C₀. In this study, the TCO-modified CC49 exhibited a shorter blood half-life (11.0 h) compared to that of the unmodified mAb (19.8 h). We attribute this to the change of isoelectric point caused by the functionalization of 7.4 Lys residues per mAb. To study the full potential of this system we selected a relatively short 24 h interval between mAb and probe administration.

### Biodistribution experiments.

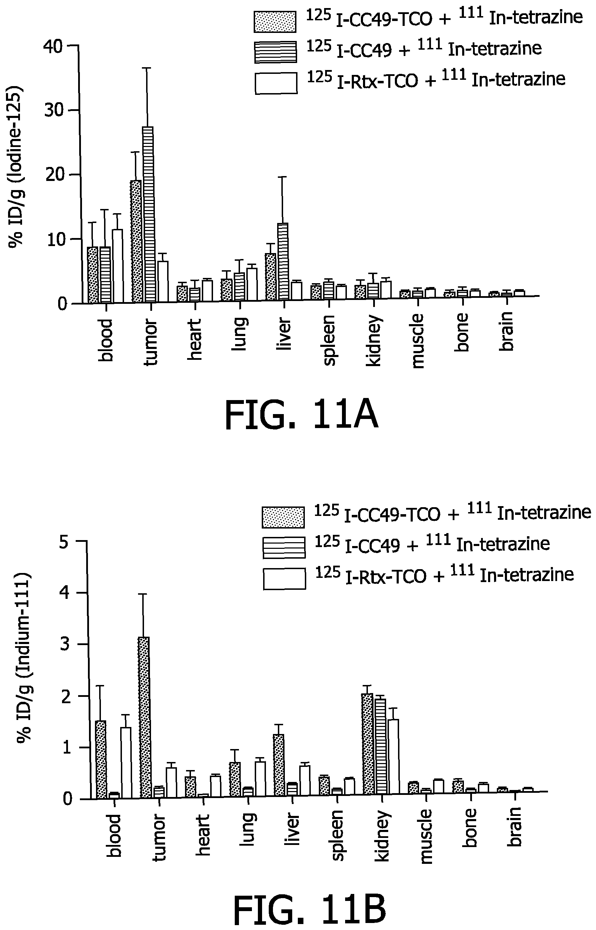

Dual isotope biodistribution experiments were performed by injecting tumor bearing mice (n=3) intravenously with ¹²⁵I-labeled mAbs (CC49-TCO, CC49 or Rtx-TCO, 100 µg/100 µL per mouse, ca. 0.2 MBq) and, 24 h later, with ¹¹¹In-tetrazine (21 µg/75 µL per mouse, ca. 0.8 MBq). Results are visualized in figure 11. Three hours after tetrazine administration, the animals were anesthetized with isoflurane and sacrificed by cervical dislocation. Blood was withdrawn by heart puncture and organs and tissues of interest were harvested, blotted dry and weighed. The radioactivity of the samples was measured in a γ-counter along with standards to determine the %ID/g. The energy windows were set to 10-80 keV and 100-510 keV for ¹²⁵I and ¹¹¹In, respectively. The sample radioactivity was measured again 3 weeks after the experiment, to check the ¹²⁵I values for potential ¹¹¹In cross-contamination. During the in vivo evaluation, the iodinated species were not subject to significant dehalogenation, as evidenced by the low amount of ¹²⁵I measured in thyroids and stomachs, and showed the typical distribution pattern of long circulating antibodies. Residual ¹²⁵I-mAbs were still detectable in blood (ca. 10%ID/g) and in blood-rich organs, such as heart and lung (which were blotted dry but not perfused with saline before counting), 27 h post injection. All species exhibited hepatobiliary clearance (¹²⁵I-activity in liver and intestine) and some kidney excretion (smaller radiometabolites). Low uptake was observed in muscle, bone and brain. In tumors, both CC49 and CC49-TCO exhibited a high accumulation with a tumor-to-blood ratio (T/B) of 3.2±2.2 and 2.8±0.8 and a tumor-to-muscle ratio (T/M) of 22.0±10.8 and 34.2±23.8, respectively. Variability in tumor uptake was observed in both groups, which is most likely due to fast and irregular tumor growth (and consequently, tumor size variation). However, the tumor accumulation of both CC49 constructs was significantly higher than for Rtx-TCO (T/B = 0.6±0.0, T/M = 4.9±0.8), indicating antigen-specific binding.

The ¹¹¹In-tetrazine distribution in the mice pre-treated with CC49-TCO or Rtx-TCO mirrored that of ¹²⁵I. In these groups, high ¹¹¹In uptake was observed in blood, heart, lung and liver, while low activity was found in spleen, muscle, bone and brain. The organs where no significant differences were found between ¹²⁵I-CC49-TCO and ¹²⁵I-Rtx-TCO uptake (blood, heart, lung, spleen, muscle, bone and brain), also did not show differences in ¹¹¹In-tetrazine accumulation. A 5.2-fold higher uptake of ¹¹¹In-tetrazine was found in the tumors containing 18.8±4.7 %ID/g ¹²⁵I-CC49-TCO compared to those containing 6.3±1.2 %ID/g ¹²⁵I-Rtx-TCO. On the other hand, almost no ¹¹¹In uptake was detected in most tissues of the group pre-treated with unmodified ¹²⁵I-CC49. Importantly, while the tumor uptake of ¹²⁵I-CC49 was the highest among the three groups, the ¹¹¹In tumor uptake in this same group was 16 times lower than in the ¹²⁵I-CC49-TCO group. Also blood retention of ¹¹¹In-tetrazine was almost undetectable in this group despite the presence of 8.7±5.9 %ID/g ¹²⁵I-CC49. Only the kidney exhibited a relatively high uptake of ¹¹¹In in all 3 groups as a consequence of radiolabeled tetrazine excretion.

The finding that the tetrazine accumulates only in organs and tissues that contain a TCO-modified species shows that a chemical reaction between these two entities occurred in vivo. We determined the yield of the DA reaction in vivo by calculating the absolute amount of TCO and tetrazine present in tissues from the %ID/g of ¹²⁵I and ¹¹¹In, respectively. In agreement with the high reactivity and selectivity between tetrazine and mAb-TCO observed in vitro, 56.7±2.0% and 52.1±3.0% of the TCO moieties present in blood and tumor, respectively, had reacted with a tetrazine probe in the group pre-treated with CC49-TCO. These yields are remarkable considering the low concentration of the components involved in the reaction (0.42±0.20 and 0.93±0.23 nmol/g in blood and tumor, respectively, for TCO and a maximum of 2.3 nmol/g in blood for the tetrazine directly after injection), the complexity of the reaction environment and the short biological half-life of the tetrazine (11.8 min).

### Imaging experiments.

Tumor-bearing mice were injected with ¹¹¹In-tetrazine (21 µg/75 µL per mouse, 20-42 MBq) 24 h after receiving 100 µg mAb (CC49-TCO, CC49 or Rtx-TCO). Approximately 1 h later, the mice were anesthetized and positioned on an animal bed equipped with a nose cone for anesthesia and a sensor for respiratory monitoring. Single photon emission computed tomography (SPECT) was performed 2 h post tetrazine injection with a four-headed multi-pinhole small animal SPECT/CT imaging system (NanoSPECT, Bioscan Inc., USA). The SPECT acquisition (1 h total) was performed with 1.4 mm diameter pinholes and a 120-140 sec acquisition time per view (24 projections). The energy window for ¹¹¹In was set at 245 keV ±15% and 171 keV ±20%. The mice were euthanized with an anesthesia overdose 3 h after tetrazine injection. Subsequently, post-mortem high resolution scans were performed with 1.0 mm diameter pinholes and a 750 sec acquisition time per view (32 projections). Prior to each SPECT session a CT scan (2 sec per projection, 360 projections) was performed to obtain anatomical information on radioactivity distribution. After the acquisition, the data was reconstructed iteratively with the manufacturer's software (InVivoScope 1.39, patch 1). Regions of interest (ROIs) were drawn manually in triplicate for tumor, liver, kidney and thigh muscle. A phantom filled with a known amount of ¹¹¹In was used to calibrate the scanner for tissue radioactivity quantification.

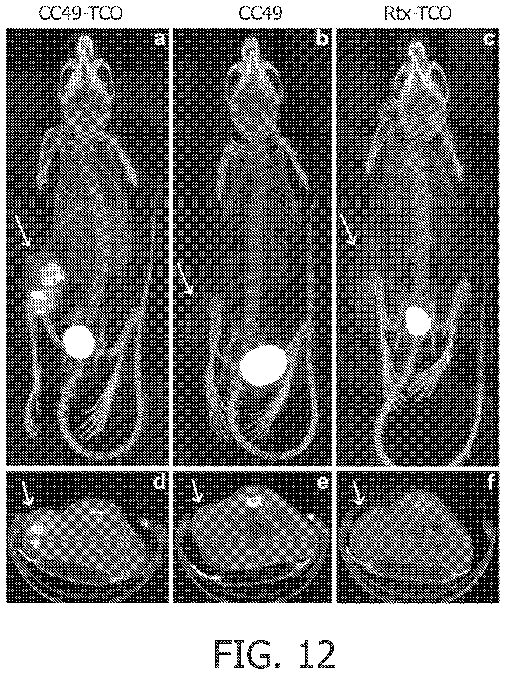

Pronounced tumor uptake of the 111In-tetrazine was demonstrated by SPECT/CT imaging of live mice up to 3 h post injection (Fig. 12A/D; tumor-to-muscle ratio (T/M)=13.1). The limited uptake in non-target tissues was attributed to reaction with residual circulating CC49-TCO. Importantly, in mice treated with unmodified CC49, the tumor could not be discriminated from the surrounding tissue (Fig. 12B/E, T/M=0.5). Almost no radioactivity was retained in blood and non-target organs as the probe was rapidly eliminated through the urinary tract, signifying its bio-orthogonality. Mice treated with TCO-modified rituximab, which lacks specificity for TAG72, showed the expected retention of 111In-tetrazine in blood and non-target organs, and a much reduced tumor accumulation (fig 12C/F).

## Claims

1. A kit for targeted medical imaging and/or therapeutics, comprising at least one Pre-targeting Probe and at least one Effector Probe, wherein the Pre-targeting Probe comprises a Primary Targeting Moiety and a first Bio-orthogonal Reactive Group, and wherein the Effector Probe comprises an Effector Moiety, such as a label or a pharmaceutically active compound, and a second Bio-orthogonal Reactive Group, wherein either of the first and second Bio-orthogonal Reactive Groups is a dienophile and the other of the first and second Bio-orthogonal Reactive Groups is a diene, wherein the dienophile is a strained 8-member ring dienophile satisfying formula (1): 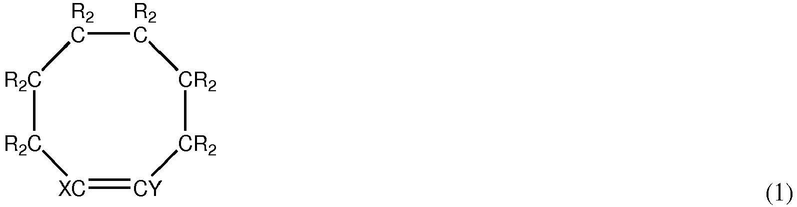 wherein each R independently denotes H, or, in at most six instances, a substituent selected from the group consisting of alkyl, O-alkyl, S-alkyl, F, Cl, Br, I, SO₂, NO₂, NR'R" with R' and R" each independently being H or alkyl, C(=O)Oalkyl, C(=O)Oaryl, CONR'R" with R' and R" each independently being H, aryl or alkyl, OCOalkyl, OCOaryl, NR'COalkyl with R' being H or alkyl, NR'COaryl with R' being or alkyl, NR'C(=O)Oalkyl with R' being H or alkyl, NR'C(=O)Oaryl with R' being H or alkyl, OCONR'alkyl with R' being H or alkyl, OCONR'aryl with R' being H or alkyl, NR'CONR"alkyl with R' and R" each independently being H or alkyl, NR'CONR"aryl with R' and R" each independently being H or alkyl, NR'CSNR"alkyl with R' and R" each independently being H or alkyl, and NR'CSNR"aryl with R' and R" each independently being H or alkyl; with at least one R comprised in a linker moiety, optionally via a spacer, to the Pre-targeting Probe or the Effector Probe wherein X and Y each independently denote H, or a substituent selected from the group consisting of alkyl, O-alkyl, S-alkyl, F, Cl, Br, I, SO₂, NO₂, and NRR' with R and R' each independently being H or alkyl, or together form a bond; and wherein the diene is selected so as to be capable of reacting with the dienophile by undergoing a Diels-Alder cycloaddition followed by a retro Diels-Alder reaction, and wherein the diene satisfies the formula 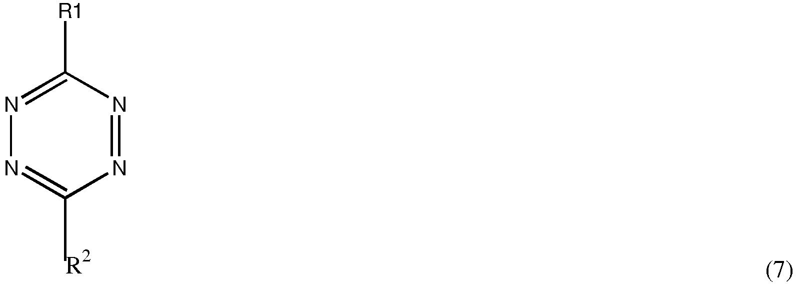 wherein R¹ and R² each independently denote a substituent selected from the group consisting of 2-pyridyl, phenyl, or phenyl substituted with one or more electron-withdrawing groups such as NO₂, CN, COOH, COOR, CONH₂, CONHR, CONR₂, CHO, COR, SO₂R, SO₂OR, NO, and Ar, wherein R is C₁-C₆ alkyl and Ar stands for an aromatic group, particularly phenyl, pyridyl, or naphthyl.

2. The kit according to claim 1, wherein said Effector Probe comprises a structural moiety capable of forming a coordination complex with a metal.

3. The kit according to claim 2, wherein said structural moiety is H4dota or H4dotma.

4. The kit according to any one of the preceding claims, wherein the Pre-targeting Probe comprises, as a primary targeting moiety, an antibody.

5. The kit according to any one of the preceding claims, wherein the Effector Probe comprises, as an effector moiety, a detectable label, preferably a contrast agent for use in imaging systems, selected from the group consisting of MRI-imageable agents, spin labels, optical labels, ultrasound- responsive agents, X-ray-responsive agents, radionuclides, FRET-type dyes, (bio)luminescent or fluorescent molecules or tags, biotin, paramagnetic imaging reagents and superparamagnetic imaging reagents.

6. The kit according to any one of the preceding claims, wherein the Effector Probe comprises, as an Effector moiety, a pharmaceutically active compound.

7. The kit according to claim 6, wherein the pharmaceutically active compound is an isotope selected from the group consisting of ²⁴Na, ³²P, ³³P, ⁴⁷Sc, ⁵⁹Fe, ⁶⁷Cu, ⁷⁶As, ⁷⁷As, ⁸⁰Br, ⁸²Br, ⁸⁹Sr, ⁹⁰Nb, ⁹⁰Y, ¹⁰³Ru, ¹⁰⁵Rh, ¹⁰⁹Pd, ¹¹¹Ag, ¹²¹Sn, ¹²⁷Te, ¹³¹I, ¹⁴⁰La, ¹⁴¹Ce, ¹⁴²Pr, ¹⁴³Pr, ¹⁴⁴Pr, ¹⁴⁹Pm, ¹⁴⁹Tb, ¹⁵¹Pm, ¹⁵³Sm, ¹⁵⁹Gd, ¹⁶¹Tb, ¹⁶⁵Dy, ¹⁶⁶Dy, ¹⁶⁶Ho, ¹⁶⁹Er, ¹⁷²Tm, ¹⁷⁵Yb, ¹⁷⁷Lu, ¹⁸⁶Re, ¹⁸⁸Re, ¹⁹⁸Au, ¹⁹⁹Au, ²¹¹At, ²¹¹Bi, ²¹²Bi, ²¹²Pb, ²¹³Bi, ²¹⁴Bi, ²²³Ra, and ²²⁵Ac.

8. An effector probe as defined in any one of claims 1 to 6, comprising an isotope selected from the group consisting of ³H, ¹¹C, ¹³N, ¹⁵O, ¹⁸F, ¹⁹ F, ⁵¹Cr, ⁵²Fe, ⁵²Mn, ⁵⁵Co, ⁶⁰Cu, ⁶¹Cu, ⁶²Zn, ⁶²Cu, ⁶³Zn, ⁶⁴Cu, ⁶⁶Ga, ⁶⁷Ga, ⁶⁸Ga, ⁷⁰As, ⁷¹As, ⁷²As, ⁷⁴As, ⁷⁵Se, ⁷⁵Br, ⁷⁶Br, ⁷⁷Br, ⁸⁰Br, ⁸²Br, ⁸²Rb, ⁸⁶Y, ⁸⁸Y, ⁸⁹Sr, ⁸⁹Zr, ⁹⁷Ru, ⁹⁹Tc, ¹¹⁰In, ¹¹¹In, ¹¹³In, ¹¹⁴In, ¹¹⁷Sn, ¹²⁰I, ¹²²Xe, ¹²³I, ¹²⁴I, ¹²⁵I, ¹⁶⁶Ho, ¹⁶⁷Tm, ¹⁶⁹Yb, ¹⁹³Pt, ¹⁹⁵Pt, ²⁰¹Tl, and ²⁰³Pb.

9. A method comprising administering a pretargeting probe as defined in any one of claims 1 to 4 to a subject and allowing the probe to circulate in the subject's system for a period of time effective to achieve binding of the primary targeting moiety to a primary target, followed by clearing non-bound agent from the body followed by the administration of an effector probe as defined in any one of claims 5 or 8, wherein the bio-orthogonal reactive groups in the pretargeting probe and in the effector probe together form the reactive partners for a [4+2] retro Diels-Alder reaction.

10. The method of claim 9, wherein said effector probe comprises a radionuclide used for imaging selected from the group consisting of ³H, ¹¹C, ¹³N, ¹⁵O, ¹⁸F, ¹⁹F, ⁵¹Cr, ⁵²Fe, ⁵²Mn, ⁵⁵Co, ⁶⁰Cu, ⁶¹Cu, ⁶²Zn, ⁶²Cu, ⁶³Zn, ⁶⁴Cu, ⁶⁶Ga, ⁶⁷Ga, ⁶⁸Ga, ⁷⁰As, ⁷¹As, ⁷²As, ⁷⁴As, ⁷⁵Se, ⁷⁵Br, ⁷⁶Br, ⁷⁷Br, ⁸⁰Br, ⁸²Br, ⁸²Rb, ⁸⁶Y, ⁸⁸Y, ⁸⁹Sr, ⁸⁹Zr, ⁹⁷Ru, ⁹⁹Tc, ¹¹⁰In, ¹¹¹In, ¹¹³In, ¹¹⁴In, ¹¹⁷Sn, ¹²⁰I, ¹²²Xe, ¹²³I, ¹²⁴I, ¹²⁵I, ¹⁶⁶Ho, ¹⁶⁷Tm, ¹⁶⁹Yb, ¹⁹³Pt, ¹⁹⁵Pt, ²⁰¹Tl, and ²⁰³Pb.

11. A kit according to claim 6 or 7, for use in targeted therapy, preferably radiation therapy.

## Patentansprüche

1. Kit für gezielte medizinische Bildgebung und/oder Therapeutika, umfassend wenigstens eine Vor-Zielende-Sonde und wenigstens eine Effektor-Sonde, wobei die Vor-Zielende-Sonde einen Primär-Zielenden-Teil und eine erste Bioorthogonale- Reaktive Gruppe umfasst, und wobei die Effektor-Sonde einen Effektor-Teil, wie eine Markierung oder eine pharmazeutisch aktive Verbindung, und eine zweite Bioorthogonale-Reaktive-Gruppe umfasst, wobei eine der ersten und zweiten Bioorthogonalen-Reaktiven-Gruppen ein Dienophil ist und die andere der ersten und zweiten Bioorthogonalen-Reaktiven Gruppen ein Dien ist, wobei das Dienophil ein gespanntes 8-gliedriges Ring-Dienophil ist, entsprechend Formel (1): 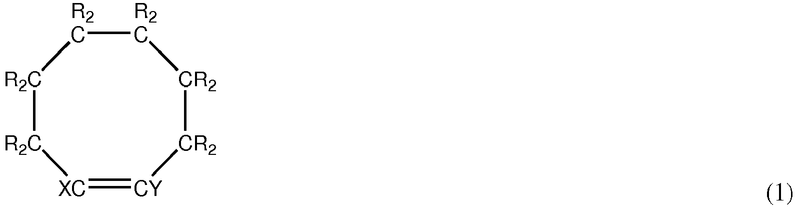 wobei jedes R unabhängig H, oder in höchstens sechs Fällen, einen Substituenten kennzeichnet, ausgewählt der aus der Gruppe , bestehend aus Alkyl, O-alkyl, S-alkyl, F, Cl, Br, I, SO₂, NO₂, NR'R", wobei R' und R" jeweils unabhängig H oder Alkyl sind, C(=O)Oalkyl, C(=O)Oaryl, CONR'R", wobei R' und R" jeweils unabhängig H, Aryl oder Alkyl sind, OCOalkyl, OCOaryl, NR'COalkyl sind, wobei R' H oder Alkyl ist, NR'COaryl, wobei R' oder Alkyl ist, NR'C(=O)Oalkyl, wobei R' H oder Alkyl ist, NR'C(=O)Oaryl, wobei R' H oder Alkyl ist, OCONR'alkyl, wobei R' H oder Alkyl ist, OCONR'aryl, wobei R' H oder Alkyl ist, NR'CONR"alkyl, wobei R' und R" jeweils unabhängig H oder Alkyl sind, NR'CONR"aryl, wobei R' und R" jeweils unabhängig H oder Alkyl sind, NR'CSNR"alkyl, wobei R' und R" jeweils unabhängig H oder Alkyl sind, und NR'CSNR"aryl, wobei R' und R" jeweils unabhängig H oder Alkyl sind; mit wenigstens einem R, umfasst in einem Linkerteil, optional über einen Spacer, an die Vor-Zielende-Sonde oder der Effektor-Sonde, wobei X und Y jeweils unabhängig H, oder einen Substituenten kennzeichnen, ausgewählt der aus der Gruppe, bestehend aus Alkyl, O-alkyl, S-alkyl, F, Cl, Br, I, SO₂, NO₂ und NRR', wobei R und R' jeweils unabhängig H oder Alkyl sind, oder zusammen eine Bindung bilden; und wobei das Dien so ausgewählt ist, dass es geeignet ist, mit dem Dienophil zu reagieren, indem es einer Diels-Alder-Cycloaddition, gefolgt von einer Retro-Diels-Alder-Reaktion unterzogen wird, und wobei das Dien der Formel entspricht 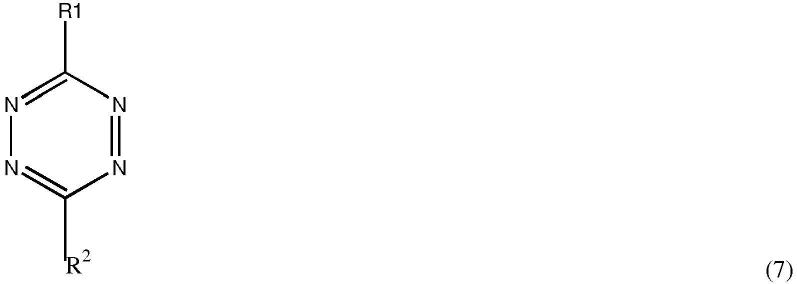 wobei R¹ und R² jeweils unabhängig einen Substituenten kennzeichnen, ausgewählt aus der Gruppe bestehend aus 2-Pyridyl, Phenyl, oder Phenyl, substituiert mit einer oder mehreren elektronenziehenden Gruppen, wie NO₂, CN, COOH, COOR, CONH₂, CONHR, CONR₂, CHO, COR, SO₂R, SO₂OR, NO, und Ar, wobei R C₁-C₆-Alkyl ist und Ar für eine aromatische Gruppe steht, insbesondere Phenyl, Pyridyl, oder Naphthyl.

2. Kit nach Anspruch 1, wobei die Effektor-Sonde einen Strukturteil umfasst, geeignet einen Koordinationskomplex mit einem Metall zu bilden.

3. Kit nach Anspruch 2, wobei der Strukturteil H4dota oder H4dotma ist.

4. Kit nach einem der vorhergehenden Ansprüche, wobei die Vor-Zielende-Sonde, als primär zielender Teil einen Antikörper umfasst.

5. Kit nach einem der vorhergehenden Ansprüche, wobei die Effektor-Sonde, als Effektor-Teil, eine nachweisbare Markierung, vorzugsweise ein Kontrastmittel zur Verwendung in Bildgebungssystemen umfasst, ausgewählt aus der Gruppe bestehend aus MRT-bildgebenden Mitteln, Spinmarkierungen, optischen Markierungen, Ultraschall-reagierende Mittel, Röntgenstrahlenreagierende Mittel, Radionuklide, FRET-Typ Farbstoffe, (bio)lumineszierende oder fluoreszierende Moleküle oder Tags, Biotin, paramagnetische Bildgebungsreagenzien und superparamagnetische Bildgebungsreagenzien.

6. Kit nach einem der vorhergehenden Ansprüche, wobei die Effektor-Sonde, als ein Effektor-Teil, eine pharmazeutisch aktive Verbindung umfasst.

7. Kit nach Anspruch 6, wobei die pharmazeutisch aktive Verbindung ein Isotop ist, ausgewählt aus der Gruppe bestehend aus ²⁴Na, ³²P, ³³P, ⁴⁷Sc, ⁵⁹Fe, ⁶⁷Cu, ⁷⁶As, ⁷⁷AS, ⁸⁰Br, ⁸²Br, ⁸⁹Sr, ⁹⁰Nb, ⁹⁰Y, ¹⁰³Ru, ¹⁰⁵Rh, ¹⁰⁹Pd, ¹¹¹Ag, ¹²¹Sn, ¹²⁷Te, ¹³¹I, ¹⁴⁰La, ¹⁴¹Ce, ¹⁴²Pr, ¹⁴³Pr, ¹⁴⁴Pr, ¹⁴⁹Pm, ¹⁴⁹Tb, ¹⁵¹Pm, ¹⁵³Sm, ¹⁵⁹Gd, ¹⁶¹Tb, ¹⁶⁵Dy, ¹⁶⁶Dy, ¹⁶⁶Ho, ¹⁶⁹Er, ¹⁷²Tm, ¹⁷⁵Yb, ¹⁷⁷Lu, ¹⁸⁶Re, ¹⁸⁸Re, ¹⁹⁸Au, ¹⁹⁹Au, ²¹¹At, ²¹¹Bi, ²¹²Bi, ²¹²Pb, ²¹³Bi, ²¹⁴Bi, ²²³Ra, und ²²⁵Ac.

8. Effektor-Sonde nach einem der Ansprüche 1 bis 6, umfassend ein Isotop, ausgewählt aus der Gruppe bestehend aus 3H, ¹¹C, ¹³N, ¹⁵O, ¹⁸F, ¹⁹F, ⁵¹Cr, ⁵²Fe, ⁵²Mn, ⁵⁵Co, ⁶⁰Cu, ⁶¹Cu, ⁶²Zn, ⁶²Cu, ⁶³Zn, ⁶⁴Cu, ⁶⁶Ga, ⁶⁷Ga, ⁶⁸Ga, ⁷⁰As, ⁷¹As, ⁷²As, ⁷⁴As, ⁷⁵Se, ⁷⁵Br, ⁷⁶Br, ⁷⁷Br, ⁸⁰Br, ⁸²Br, 82Rb, ⁸⁶Y, ⁸⁸Y, ⁸⁹Sr, ⁸⁹Zr, ⁹⁷Ru, ⁹⁹Tc, ¹¹⁰In, ¹¹¹In, ¹¹³In, ¹¹⁴In, ¹¹⁷Sn, ¹²⁰I, ¹²²Xe, ¹²³I, ¹²⁴I, ¹²⁵I, ¹⁶⁶Ho, ¹⁶⁷Tm, ¹⁶⁹Yb, ¹⁹³Pt, ¹⁹⁵Pt, ²⁰¹Tl, und ²⁰³Pb.

9. Verfahren, umfassend Verabreichen einer vorzielenden Sonde, wie definiert in einem der Ansprüche 1 bis 4 an ein Subjekt und Zulassen der Sonde im System des Subjekts für einen Zeitraum zu zirkulieren, wirksam, um das Binden des primär zielenden Teils an ein primäres Ziel zu erreichen, gefolgt vom Klären nicht-gebundenen Mittels aus dem Körper, gefolgt von der Verabreichung einer Effektor-Sonde, wie definiert in einem der Ansprüche 5 oder 8, wobei die bioorthogonalen reaktiven Gruppen in der vorzielenden Sonde und in der Effektor-Sonde zusammen die reaktiven Partner für eine [4+2] Retro-Diels-Alder-Reaktion bilden.

10. Verfahren nach Anspruch 9, wobei die Effektor-Sonde ein Radionuklid, verwendet zur Bildgebung, umfasst, ausgewählt aus der Gruppe, bestehend aus ³H, ¹¹C, 13N, 150, ¹⁸F, ¹⁹F, ⁵¹Cr, ⁵²Fe, ⁵²Mn, 55Co, ⁶⁰Cu, ⁶¹Cu, ⁶²Zn, ⁶²Cu, ⁶³Zn, ⁶⁴Cu, ⁶⁶Ga, ⁶⁷Ga, ⁶⁸Ga, ⁷⁰As, ⁷¹As, ⁷²As, ⁷⁴As, ⁷⁵Se, ⁷⁵Br, ⁷⁶Br, ⁷⁷Br, ⁸⁰Br, ⁸²Br, ⁸²Rb, ⁸⁶Y, ⁸⁸Y, ⁸⁹Sr, ⁸⁹Zr, ⁹⁷Ru, ⁹⁹Tc, ¹¹⁰In, ¹¹¹In, ¹¹³In, ¹¹⁴In, ¹¹⁷Sn, ¹²⁰I, ¹²²Xe, ¹²³I, ¹²⁴I, ¹²⁵I, ¹⁶⁶Ho, ¹⁶⁷Tm, ¹⁶⁹Yb, ¹⁹³Pt, ¹⁹⁵Pt, ²⁰¹Tl, und ²⁰³Pb.

11. Kit nach Anspruch 6 oder 7, zur Verwendung in einer gezielten Therapie, vorzugsweise Strahlungstherapie.

## Revendications

1. Trousse pour une imagerie médicale et/ou des agents thérapeutiques ciblés, comprenant au moins une sonde de pré-ciblage et au moins une sonde effectrice, dans laquelle la sonde de pré-ciblage comprend un fragment de ciblage primaire et un premier groupe réactif bio-orthogonal, et dans laquelle la sonde effectrice comprend un fragment effecteur, tel qu'une étiquette ou un composé pharmaceutiquement actif, et un deuxième groupe réactif bio-orthogonal, dans laquelle l'un ou l'autre parmi les premier et deuxième groupes réactifs bio-orthogonaux est un diénophile et l'autre parmi les premier et deuxième groupes réactifs bio-orthogonaux est un diène, dans laquelle le diénophile est un diénophile cyclique à 8 chaînons contraint répondant à la formule (1) : 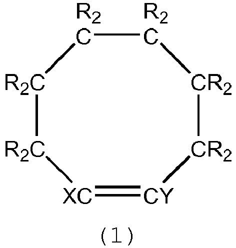 dans laquelle chaque R représente indépendamment H ou, dans au plus six cas, un substituant choisi dans l'ensemble constitué par alkyle, O-alkyle, S-alkyle, F, Cl, Br, I, SO₂, NO₂, NR'R" où chacun de R' et R" est indépendamment H ou un alkyle, C(=O)O-alkyle, C(=O)O-aryle, CONR'R" où chacun de R' et R" est indépendamment H ou un aryle ou alkyle, OCO-alkyle, OCO-aryle, NR'CO-alkyle où R' est H ou un alkyle, NR'CO-aryle où R' est H ou un alkyle, NR'C(=O) O-alkyle où R' est H ou un alkyle, NR'C (=O) O-aryle où R' est H ou un alkyle, OCONR'-alkyle où R' est H ou un alkyle, OCONR'-aryle où R' est H ou un alkyle, NR'CONR"-alkyle où chacun de R' et R" est indépendamment H ou un alkyle, NR'CONR"-aryle où chacun de R' et R" est indépendamment H ou un alkyle, NR'CSNR"-alkyle où chacun de R' et R" est indépendamment H ou un alkyle, et NR'CSNR"-aryle où chacun de R' et R" est indépendamment H ou un alkyle ; au moins un R étant compris dans un fragment lieur, éventuellement via un écarteur, à la sonde de pré-ciblage ou la sonde effectrice où chacun de X et Y représente indépendamment H, ou un substituant choisi dans l'ensemble constitué par alkyle, O-alkyle, S-alkyle, F, Cl, Br, I, SO₂, NO₂, et NRR' où chacun de R et R' est indépendamment H ou un alkyle, ou ensemble forment une liaison ; et dans laquelle le diène est choisi de façon à être capable de réagir avec le diénophile en subissant une cycloaddition de Diels-Alder suivie d'une rétroaction de Diels-Alder, et dans laquelle le diène répond à la formule 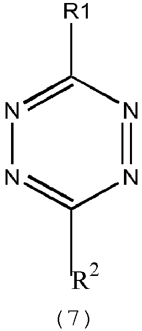 dans laquelle chacun de R¹ et R² représente indépendamment un substituant choisi dans l'ensemble constitué par 2-pyridyle, phényle, ou phényle substitué par un ou plusieurs groupes extracteurs d'électrons tels que NO₂, CN, COOH, COOR, CONH₂, CONHR, CONR₂, CHO, COR, SO₂R, SO₂OR, NO et Ar, où R est un alkyle en C₁ à C₆ et Ar représente un groupe aromatique, en particulier phényle, pyridyle ou naphtyle.

2. Trousse selon la revendication 1, dans laquelle ladite sonde effectrice comprend un fragment structurel capable de former un complexe de coordination avec un métal.

3. Trousse selon la revendication 2, dans laquelle ledit fragment structurel est H4dota ou H4dotma.

4. Trousse selon l'une quelconque des revendications précédentes, dans laquelle la sonde de pré-ciblage comprend, en tant que fragment de ciblage primaire, un anticorps.

5. Trousse selon l'une quelconque des revendications précédentes, dans laquelle la sonde effectrice comprend, en tant que fragment effecteur, un marqueur détectable, de préférence un agent de contraste pour une utilisation dans des systèmes d'imagerie, choisi dans l'ensemble constitué par les agents pouvant former une image IRM, les marqueurs de spin, les marqueurs optiques, les agents sensibles aux ultrasons, les agents sensibles aux rayons X, les radionucléides, les colorants de type FRET, les molécules ou étiquettes (bio)luminescentes ou fluorescentes, la biotine, les réactifs d'imagerie paramagnétique et les réactifs d'imagerie superparamagnétique.

6. Trousse selon l'une quelconque des revendications précédentes, dans laquelle la sonde effectrice comprend, en tant que fragment effecteur, un composé pharmaceutiquement actif.

7. Trousse selon la revendication 6, dans laquelle le composé pharmaceutiquement actif est un isotope choisi dans l'ensemble constitué par ²⁴Na, ³²P, ³³P, ⁴⁷Sc, ⁵⁹Fe₅ ⁶⁷Cu, ⁷⁶As, ⁷⁷As, ⁸⁰Br, ⁸²Br, ⁸⁹Sr, ⁹⁰Nb, ⁹⁰Y, ¹⁰³Ru, ¹⁰⁵Rh, ¹⁰⁹Pd, ¹¹¹Ag, ¹²¹Sn, ¹²⁷Te, ¹³¹I, ¹⁴⁰La, ¹⁴¹Ce, ¹⁴²Pr, ¹⁴³Pr, ¹⁴⁴Pr, ¹⁴⁹Pm, ¹⁴⁹Tb, ¹⁵¹Pm, ¹⁵³Sm, ¹⁵⁹Gd, ¹⁶¹Tb, ¹⁶⁵Dy, ¹⁶⁶Dy, ¹⁶⁶Ho, ¹⁶⁹Er, ¹⁷²Tm, ¹⁷⁵Yb, ¹⁷⁷Lu, ¹⁸⁶Re, ¹⁸⁸Re, ¹⁹⁸Au, ¹⁹⁹Au, ²¹¹At, ²¹¹Bi, ²¹²Bi, ²¹²Pb, ²¹³Bi, ²¹⁴Bi, ²²³Ra, et ²²⁵Ac.

8. Sonde effectrice telle que définie dans l'une quelconque des revendications 1 à 6, comprenant un isotope choisi dans l'ensemble constitué par ³H, ¹¹C, ¹³N, ¹⁵O, ¹⁸F, ¹⁹F, ⁵¹Cr, ⁵²Fe, ⁵²Mn, ⁵⁵Co, ⁶⁰Cu, ⁶¹Cu, ⁶²Zn, ⁶²Cu, ⁶³Zn, ⁶⁴Cu, ⁶⁶Ga, ⁶⁷Ga, ⁶⁸Ga, ⁷⁰As, ⁷¹As, ⁷²As, ⁷⁴As, ⁷⁵Se, ⁷⁵Br, ⁷⁶Br, ⁷⁷Br, ⁸⁰Br, ⁸²Br, ⁸²Rb, ⁸⁶Y, ⁸⁸Y, ⁸⁹Sr, ⁸⁹Zr, ⁹⁷Ru, ⁹⁹Tc, ¹¹⁰In, ¹¹¹In, ¹¹³In, ¹¹⁴In, ¹¹⁷Sn, ¹²⁰I, ¹²²Xe, ¹²³I, ¹²⁴I, ¹²⁵I, ¹⁶⁶Ho, ¹⁶⁷Tm, ¹⁶⁹Yb, ¹⁹³Pt, ¹⁹⁵Pt, ²⁰¹Tl, et ²⁰³Pb.

9. Procédé comprenant l'administration d'une sonde de pré-ciblage telle que définie dans l'une quelconque des revendications 1 à 4 à un sujet et le fait de laisser la sonde circuler dans le système du sujet pendant une période de temps efficace pour que soit obtenue la liaison du fragment de ciblage primaire à une cible primaire, opération suivie de la clairance de l'agent non lié hors du corps, suivie de l'administration d'une sonde effectrice telle que définie dans l'une quelconque des revendications 5 et 8, dans lequel les groupes réactifs bio-orthogonaux dans la sonde de pré-ciblage et dans la sonde effectrice forment ensemble les partenaires réactifs pour une rétroaction de Diels-Alder [4+2].

10. Procédé selon la revendication 9, dans lequel ladite sonde effectrice comprend un radionucléide utilisé pour l'imagerie, choisi dans l'ensemble constitué par ³H, ¹¹C, ¹³N, ¹⁵O, ¹⁸F, ¹⁹F, ⁵¹Cr, ⁵²Fe, ⁵²Mn, ⁵⁵Co, ⁶⁰Cu, ⁶¹Cu, ⁶²Zn, ⁶²Cu, ⁶³Zn, ⁶⁴Cu, ⁶⁶Ga, ⁶⁷Ga, ⁶⁸Ga, ⁷⁰As, ⁷¹As, ⁷²As, ⁷⁴As, ⁷⁵Se, ⁷⁵Br, ⁷⁶Br, ⁷⁷Br, ⁸⁰Br, ⁸²Br, ⁸²Rb, ⁸⁶Y, ⁸⁸Y, ⁸⁹Sr, ⁸⁹Zr, ⁹⁷Ru, ⁹⁹Tc, ¹¹⁰In, ¹¹¹In, ¹¹³In, ¹¹⁴In, ¹¹⁷Sn, ¹²⁰I, ¹²²Xe, ¹²³I, ¹²⁴I, ¹²⁵I, ¹⁶⁶Ho, ¹⁶⁷Tm, ¹⁶⁹Yb, ¹⁹³Pt, ¹⁹⁵Pt, ²⁰¹Tl, et ²⁰³Pb.

11. Trousse selon la revendication 6 ou 7, pour une utilisation en thérapie ciblée, de préférence en radiothérapie.
